# EUROPÄISCHE PATENTSCHRIFT

(11) **EP 2 340 502 B1**
(45) Veröffentlichungstag und Bekanntmachung des Hinweises auf die Patenterteilung: **23.03.2016**
(21) Anmeldenummer: 09783152.3
(22) Anmeldetag: 18.09.2009
(51) Int. Cl.: G06F 21/34, H04L 9/08, H04L 9/32, G06Q 50/22

(54) **DATENVERARBEITUNGSSYSTEM ZUR BEREITSTELLUNG VON BERECHTIGUNGSSCHLÜSSELN**
DATA PROCESSING SYSTEM FOR PROVIDING AUTHORIZATION KEYS
SYSTÈME DE TRAITEMENT DE DONNÉES POUR PRÉPARER DES CLÉS D'AUTORISATION

(30) Priorität: 10.10.2008 DE 202008013415 U
(43) Veröffentlichungstag der Anmeldung: 06.07.2011
(73) Patentinhaber: CompuGroup Holding AG, 56070 Koblenz (DE)
(72) Erfinder: SPALKA, Adrian, 56068 Koblenz (DE); LEHNHARDT, Jan, 56068 Koblenz (DE); SCHMID, Michael, 55437 Ockenheim (DE)
(74) Vertreter: Richardt Patentanwälte PartG mbB
(86) Internationale Anmeldenummer: PCT/EP2009/062093
(87) Internationale Veröffentlichungsnummer: WO 2010/040629

(56) Entgegenhaltungen:
- WO-A1-2006/029820
- US-A1- 2004 172 538
- SCHNEIER B ED - SCHNEIER B: "APPLIED CRYPTOGRAPHY, PASSAGE", 1. Januar 1996 (1996-01-01), APPLIED CRYPTOGRAPHY, PROTOCOLS, ALGORITHMS, AND SOURCE CODE IN C, JOHN WILEY & SONS, INC, NEW YORK, PAGE(S) 173 - 175, XP000864254, ISBN: 978-0-471-11709-4 Seite 173 - Seite 175
- OWENS L ET AL: "An Identity Based Encryption System", PROCEEDINGS OF THE 3RD INTERNATIONAL SYMPOSIUM ON PRINCIPLES AND PRACTICE OF PROGRAMMING IN JAVA (PPPJ '04). LAS VEGAS, NEVADA, JUNE 16 20040616; 20040616 - 20040618, 16. Juni 2004 (2004-06-16), Seiten 154-159, XP040022545, ISBN: 978-1-59593-171-9
- Schneier, Bruce: "Applied Cryptography", 1. Januar 1996 (1996-01-01), JOHN WILEY & SONS, INC, NEW YORk, XP002619016, Seiten 52-53, Seite 52 - Seite 53

## Beschreibung

Die Erfindung betrifft ein Computerprogrammprodukt mit von einem Prozessor ausführbaren Instruktionen zur Durchführung von Verfahrensschritten zur Entschlüsselung eines Datenobjekts und ein Datenverarbeitungssystem.

Die elektronische Gesundheitskarte, abgekürzt eGK, soll in Zukunft die Krankenversicherungskarte in Deutschland ersetzen. Ziel ist es dabei, eine Datenübermittlung zwischen medizinischen Leistungserbringern, Krankenkassen, Apotheken und Patienten in Zukunft kostengünstiger zu gestalten, zu vereinfachen und zu beschleunigen. Dazu gehört auch unter anderem die Ermöglichung eines Zugriffs auf einen elektronischen Arztbrief, eine elektronische Krankenakte sowie ein elektronisches Rezept mit Hilfe der elektronischen Gesundheitskarte.

Beispielsweise können somit medizinische Datenobjekte (MDOs) wie ein elektronischer Arztbrief, eine elektronische Krankenakte oder ein elektronisches Rezept verschlüsselt und digital signiert auf einem zentralen Server gespeichert werden. Eine Verschlüsselung erfolgt dabei vorzugsweise über einen symmetrischen Schlüssel, der für jedes neue medizinische Datenobjekt einer elektronischen Krankenakte wie z.B. ein elektronischer Arztbrief oder ein elektronisches Rezept individuell zufällig erzeugt wird. Der symmetrische Schlüssel selbst wird nach seiner Erstellung beispielsweise mit einem öffentlichen Schlüssel verschlüsselt und zusammen mit den verschlüsselten medizinischen Datenobjekten auf dem zentralen Server abgelegt. Dieser zur Verschlüsselung verwendete öffentliche Schlüssel bildet dabei zusammen mit einem privaten Schlüssel, welcher auf der elektronischen Gesundheitskarte abgespeichert ist, ein kryptografisches asymmetrisches Schlüsselpaar. Damit ist gewährleistet, dass ausschließlich unter Verwendung des geheimen Gesundheitskartenschlüssels ein Zugriff auf die verschlüsselten medizinischen Datenobjekte möglich ist. Bei einem solchen Zugriff erfolgt zunächst eine Entschlüsselung des verschlüsselten symmetrischen Schlüssels mittels des geheimen Gesundheitskartenschlüssels, woraufhin dann mit dem entschlüsselten symmetrischen Schlüssel eine weitere Entschlüsselung der medizinischen Datenobjekte möglich ist. Wurde bei der Erstellung eines MDOs auch eine digitale Signatur mit dem geheimen Gesundheitskartenschlüssel erzeugt, so kann anschließend die Integrität des MDOs und die Authentizität des MDO-Erzeugers über die digitale Signatur verifiziert werden.

Beispielsweise offenbart die DE 10 2004 051 296 B3 ein Verfahren zur Speicherung von Daten und zur Abfrage von Daten sowie entsprechende Computerprogrammprodukte. Eine personalisierte Chipkarte ermöglicht die Speicherung einer virtuellen Patientenakte auf einem Datenserver. Unter Verwendung der Chipkarte können Daten, wie zum Beispiel ein MDO einer Patientenakte, von einem Praxis-EDV-System einer Arztpraxis verschlüsselt und digital signiert an einen Datenserver übertragen werden.

Aus der DE 102 58 769 A1 ist eine weitere Anwendung von Chipkarten für Patientendaten bekannt.

Bei der Verwendung der elektronischen Gesundheitskarte ergibt sich die Problematik, dass im Falle beispielsweise eines Krankenkassenwechsels und der damit verbundenen Ausgabe einer neuen elektronischen Gesundheitskarte mit entsprechenden neuen asymmetrischen Schlüsselpaaren ein problemloser Zugriff auf eine elektronische Krankenakte nicht mehr möglich ist, welche zuvor unter Verwendung der alten elektronischen Gesundheitskarte verschlüsselt wurde.

Die US 2004/0172538 A1 zeigt eine Datenspeichervorrichtung mit einer Verschlüsselungseinheit, welche Daten unter Verwendung eines Schlüssels verschlüsseln kann, wobei der Schlüssel unter Verwendung eines Passwortes erzeugt wird. Der Schlüssel kann unter Verwendung eines Master-Schlüssels ebenfalls verschlüsselt werden.

Der Erfindung liegt demgegenüber die Aufgabe zugrunde, ein verbessertes Computerprogrammprodukt zur Durchführung von Verfahrensschritten zur Entschlüsselung eines Datenobjekts und ein verbessertes Datenverarbeitungssystem zu schaffen.

Die der Erfindung zugrunde liegenden Aufgaben werden jeweils mit den Merkmalen der unabhängigen Schutzansprüche gelöst. Bevorzugte Ausführungsformen der Erfindung sind in den abhängigen Schutzansprüchen angegeben.

Die Erfindung betrifft ein Computerprogrammprodukt mit von einem Prozessor ausführbaren Instruktionen zur Durchführung von Verfahrensschritten zur Entschlüsselung eines Datenobjekts, wobei das Datenobjekt über einen initialen ersten Berechtigungsschlüssel entschlüsselbar ist, wobei der initiale erste Berechtigungsschlüssel zusammen mit einem initialen zweiten Berechtigungsschlüssel ein initiales asymmetrisches kryptografisches Schlüsselpaar bildet, wobei das initiale asymmetrische Schlüsselpaar Teil einer Schlüsselpaarsequenz ist. Das Verfahren umfasst dabei die Schritte des Zugriffs auf einen ersten Berechtigungsschlüssel, wobei der erste Berechtigungsschlüssel zusammen mit einem zweiten Berechtigungsschlüssel ein asymmetrisches kryptografisches Schlüsselpaar bildet, wobei das asymmetrische Schlüsselpaar Teil der Schlüsselpaarsequenz ist. Daraufhin erfolgt das Abrufen eines Chiffrats, wobei das Chiffrat dem asymmetrischen kryptografischen Schlüsselpaar zugeordnet ist, wobei das Chiffrat den initialen ersten Schlüssel verschlüsselt mit dem zweiten Berechtigungsschlüssel umfasst. Es erfolgt das Entschlüsseln des verschlüsselten initialen ersten Schlüssels mit dem ersten Berechtigungsschlüssel und das Entschlüsseln des verschlüsselten Datenobjekts über den entschlüsselten initialen ersten Schlüssel.

Zusätzlich umfasst das Computerprogrammprodukt ferner Instruktionen zur Durchführung des Schritts einer Signaturüberprüfung des Datenobjekts, wobei die Signaturüberprüfung die Schritte umfasst des Lesens einer dem Datenobjekt zugeordneten Signatur und der Verifizierung der Signatur des Datenobjekts, wobei die Verifikation mit dem zweiten Berechtigungsschlüssel erfolgt.

Dieses Verfahren hat den Vorteil, dass eine Entschlüsselung des verschlüsselten Datenobjektes unter Verwendung eines beliebigen asymmetrischen Schlüsselpaares durchgeführt werden kann, welches Teil der Schlüsselpaarsequenz ist. Es spielt hierbei keine Rolle, mit welchem asymmetrischen Schlüsselpaar des Satzes von asymmetrischen Schlüsselpaaren der Schlüsselpaarsequenz zuvor eine Verschlüsselung des Datenobjekts erfolgt ist. Abhängig von dem zur Entschlüsselung nun verwendeten asymmetrischen kryptografischen Schlüsselpaar wird ein entsprechendes Chiffrat abgerufen, mittels welchem der initiale erste Schlüssel extrahiert werden kann, unter Verwendung dessen schließlich das Datenobjekt entschlüsselt werden kann.

Die Durchführung einer zusätzlichen Signaturüberprüfung im Falle einer zuvor erfolgten Signierung des Datenobjekts mit dem ersten Berechtigungsschlüssel hat den Vorteil, dass hiermit verifiziert werden kann, dass das Datenobjekt seit dem ursprünglichen Verschlüsselungsvorgang nicht unbefugt modifiziert wurde.

Die Durchführung der genannten Verfahrensschritte hat also den Vorteil, dass mit beliebigen asymmetrischen kryptografischen Schlüsselpaaren innerhalb einer Sequenz von Schlüsselpaaren gewährleistet ist, dass Datenobjekte, welche unter Verwendung eines der asymmetrischen kryptografischen Schlüsselpaare der Sequenz von Schlüsselpaaren verschlüsselt wurden, wieder entschlüsselt werden können. Zur Entschlüsselung ist hierfür das zur ursprünglichen Verschlüsselung verwendete "Original" Schlüsselpaar nicht notwendig.

Bevorzugterweise wird so vorgegangen, dass neue Datenobjekte grundsätzlich mit dem initialen zweiten Berechtigungsschlüssel verschlüsselt werden. Unter Verwendung eines beliebigen Schlüsselpaares der Sequenz von asymmetrischen kryptografischen Schlüsselpaaren kann daraufhin durch entsprechenden Zugriff auf das Chiffrat, welches dem jeweiligen Schlüsselpaar zugeordnet ist, der initiale erste Berechtigungsschlüssel bereitgestellt werden, mit welchem wiederum eine Entschlüsselung des Datenobjekts möglich ist.

Zur Durchführung der genannten Verfahrensschritte ist zur Verwendung eines neuen bzw. weiteren asymmetrischen kryptografischen Schlüsselpaares für Ver- und Entschlüsselungsvorgänge von Datenobjekten somit lediglich Voraussetzung, dass ein entsprechender Benutzer im Besitz des asymmetrischen kryptografischen Schlüsselpaares ist, welches in der Sequenz von Schlüsselpaaren dem neuen Schlüsselpaar unmittelbar vorangeht. In einem praktischen Beispiel von Gesundheitskarten würde dies damit bedeuten, dass ein Besitzer einer neuen elektronischen Gesundheitskarte zu deren "Aktivierung" lediglich einmalig seine zuvor verwendete alte elektronische Gesundheitskarte zu einem entsprechenden Gesundheitsdienstleister oder allgemein einer vertrauenswürdigen Stelle mitbringt. Unter Verwendung der alten und der neuen elektronischen Gesundheitskarte wird daraufhin das neue Chiffrat erzeugt, mittels welchem es möglich ist, sowohl mit der alten als auch mit der neuen elektronischen Gesundheitskarte auf verschlüsselte Datenobjekte sicher zuzugreifen.

Nach einer Ausführungsform der Erfindung wird das Chiffrat in einer Datenbank gespeichert. Beispielsweise kann hier eine Datenbank einer zentralen vertrauenswürdigen Stelle zum Einsatz kommen.
Nach einer weiteren Ausführungsform der Erfindung umfasst das Computerprogrammprodukt ferner Instruktionen zur Durchführung des Schritts des Empfangens einer dem asymmetrischen kryptografischen Schlüsselpaar zugeordneten Schlüsselpaarkennung, wobei das Chiffrat anhand der Schlüsselpaarkennung abgerufen wird. Beispielsweise wird das Chiffrat von einer Datenbank abgerufen. Alternativ dazu ist es zum Beispiel möglich, dass das asymmetrische Schlüsselpaar zusammen mit dem Chiffrat auf einem tragbaren Datenträger, wie zum Beispiel einer Chipkarte, gespeichert ist.

Nach einer weiteren Ausführungsform der Erfindung ist das Datenobjekt mit einem symmetrischen Datenobjektschlüssel verschlüsselt, wobei der symmetrische Datenobjektschlüssel mit dem initialen zweiten Berechtigungsschlüssel verschlüsselt ist, wobei das Entschlüsseln des verschlüsselten Datenobjekts in diesem Fall den weiteren Schritt umfasst des Entschlüsselns des verschlüsselten symmetrischen Datenobjektschlüssels mit dem entschlüsselten initialen ersten Berechtigungsschlüssel und dem Entschlüsseln des verschlüsseltes Datenobjekts mit dem entschlüsselten symmetrischen Schlüssel.

Nach einer weiteren Ausführungsform der Erfindung umfasst der Zugriff auf den ersten Berechtigungsschlüssel den Schritt des Empfangens einer Benutzeridentifikation und einer der Benutzeridentifikation zugeordneten Benutzerkennung. Dem folgen das Abrufen eines der Benutzeridentifikation zugeordneten Zufallswerts von einer weiteren Datenbank und das Berechnen des ersten Berechtigungsschlüssels, wobei in die Berechnung der Zufallswert und die Benutzerkennung eingehen.

Nach einer weiteren Ausführungsform der Erfindung umfasst die Signaturüberprüfung ferner den Schritt des Berechnens des zweiten Berechtigungsschlüssels aus dem ersten Berechtigungsschlüssel mittels eines asymmetrischen kryptografischen Schlüsselerzeugungsverfahrens, wobei der erste und der zweite Berechtigungsschlüssel das asymmetrische kryptografische Schlüsselpaar bilden.

Nach einer weiteren Ausführungsform der Erfindung wird der Zufallswert über eine sichere Kommunikationsverbindung von der weiteren Datenbank abgerufen. Dabei ist es möglich, dass die Datenbank, von welcher das Chiffrat abgerufen wird, und von welcher der Zufallswert abgerufen wird, identisch ist.

Nach einer weiteren Ausführungsform der Erfindung ist der Zufallswert verschlüsselt in der weiteren Datenbank abgespeichert.

Nach einer weiteren Ausführungsform der Erfindung handelt es sich bei dem Datenobjekt um ein medizinisches Datenobjekt.

Ferner kann das Computerprogrammprodukt ferner Instruktionen zur Durchführung des Schrittes des Erzeugens des weiteren asymmetrischen kryptografischen Schlüsselpaars umfassen, wobei die Instruktionen die folgenden Schritte umfassen:
1. Empfang einer eindeutigen Benutzeridentifikation id und einer der Benutzeridentifikation zugeordneten beliebig wählbaren Benutzerkennung pw.
2. Abbildung der Benutzerkennung auf einen Wert durch eine Funktion g. Bei der Funktion g kann es sich um die Identitätsfunktion oder eine nicht triviale Funktion handeln. Unter dem Aspekt der Sicherheit und Vertraulichkeit ist g vorzugsweise als eine kollisionsfreie Einwegfunktion gewählt (sog. one-way Funktion), wie z.B. eine kryptografische Hashfunktion.
3. Erzeugung eines Zufallswertes z.
4. Berechnung des weiteren ersten Datenobjektschlüssels durch Anwendung einer Funktion f auf g(Benutzerkennung) und z. Beispielsweise werden g(Benutzerkennung), d.h. das Ergebnis der Anwendung der Funktion g auf die Benutzerkennung, und z miteinander verkettet und die Funktion f wird auf das Ergebnis dieser Verkettung angewendet. Z.B. kann feine kryptografische Hash-Funktion sein, die auf die Konkatenation des Hash-Wertes der Benutzerkennung und des Zufallswertes z angewendet wird.
5. Berechnung des weiteren zweiten Datenobjektschlüssels aus dem weiteren ersten Datenobjektschlüssel, wobei der weitere erste und der weitere zweite Datenobjektschlüssel ein asymmetrisches kryptografisches Schlüsselpaar bilden. Beispielsweise kann:
   - für elliptische Kurven der weitere zweite Datenobjektschlüssel, der ein Punkt auf der elliptischen Kurve ist, durch Multiplikation des ersten Datenobjektschlüssels, der eine ganze Zahl darstellt, mit dem Basispunkt aus den Domainparametern berechnet werden.
   - für RSA wird der weitere zweite Datenobjektschlüssel (eine ganze Zahl) derart berechnet, dass er mit dem ersten Datenobjektschlüssel (ebenfalls eine ganze Zahl) eine im RSA-Verfahren definierte Kongruenzrelation erfüllt.

Die Durchführung der genannten Verfahrensschritte hat den Vorteil, dass hier asymmetrische kryptografische Schlüsselpaare erzeugt werden können, wobei dies unter Verwendung einer beliebig wählbaren Benutzerkennung erfolgt. Die Benutzerkennung selbst geht in den Berechnungsalgorithmus für den weiteren ersten und weiteren zweiten Datenobjektschlüssel ein.

Im Falle dessen, dass das erfindungsgemäße Computerprogrammprodukt im Rahmen der elektronischen Gesundheitskarte eingesetzt wird, ist Benutzer in der Lage, einen Zugriff auf seine Patientendaten entweder unter Verwendung seiner elektronischen Gesundheitskarte, oder aber unter Verwendung seiner selbst gewählten Benutzerkennung durchzuführen. Entscheidet sich ein Benutzer, zusätzlich neben seiner elektronischen Gesundheitskarte auch noch die Möglichkeit eines passwortgestützten Zugriffs auf seine Patientendaten zu ermöglichen, so wird auch hier vorzugsweise von einer vertrauenswürdigen Stelle einmalig nach Eingabe der Benutzerkennung das entsprechende weitere asymmetrische kryptografische Schlüsselpaar erzeugt und unter Verwendung der zusätzlich im Besitz des Patienten befindlichen elektronischen Gesundheitskarte das Chiffrat erstellt, sodass auf Datenobjekte, welche zuvor unter Verwendung der elektronischen Gesundheitskarte chiffriert wurden, auch mittels der weiter gewählten Benutzerkennung zugegriffen werden kann, wobei dies auch in umgekehrter Reihenfolge gilt.

Damit unterscheidet sich dieses Verfahren zur Erzeugung eines asymmetrischen kryptografischen Schlüsselpaares von gängigen Schlüsselerzeugungsverfahren, bei welchen nach heutigem Stand der Technik lediglich eine Zuordnung einer beliebig wählbaren Benutzerkennung zu einem zugehörigen erzeugten kryptografischen Schlüsselpaar möglich ist, nicht hingegen eine funktionale Berechung von Schlüsselpaaren unter Verwendung der beliebig wählbaren Benutzerkennung selbst, bei der die permanente Speicherung der Zuordnung der Benutzerkennung zum Schlüssel entfällt.

Bei bisher üblichen Verfahren wird eine beliebig gewählte Benutzerkennung oder deren Abbild in einer Tabelle gespeichert und öffentlichen und privaten Schlüsseln eindeutig zugeordnet, wobei lediglich durch administrative und/oder juristische Regelungen fest-gelegt wird, dass unbefugte Personen keinen Zugriff auf den privaten Schlüssel haben dürfen. Diese Vorgehensweise beeinträchtigt die Sicherheit erheblich: Bekommt eine unbefugte Person oder auch eine staatliche Stelle aufgrund von diversen Überwachungsgesetzen Zugriff auf die Datenbank, welche die Passwörter den öffentlichen und privaten Schlüsseln zuordnet, so ist diese Person oder Organisation sofort in der Lage, durch Zugriff auf diese einzelne schlüsselverwaltende Institution Zugriff auf alle Datenobjekte einer Person zu erhalten.

Damit hat diese Vorgehensweise den Vorteil, dass nebst der Möglichkeit einer beliebig wählbaren Benutzerkennung keine zentrale Instanz in Besitz der Kombination von Benutzerkennung (z.B. Passwort) und Schlüsselpaaren gelangen kann. Der weitere erste Datenobjektschlüssel lässt sich lediglich unter Kenntnis eines Zufallswertes und der Benutzerkennung berechnen. Die Erzeugung des weiteren zweiten Datenobjektschlüssels erfordert ebenfalls die Kenntnis des Zufallswerts und der Benutzerkennung, wobei die Benutzerkennung vorzugsweise ausschließlich in geheimer Weise in Kenntnis des entsprechenden Benutzers steht. Damit ist es zum Beispiel nicht mehr möglich, durch Beschlagnahmung oder Diebstahl von zentralen Datenbankservern Zugriff auf Datenobjektschlüssel und damit auf verschlüsselte Daten ohne aktives Mithelfen jener Personen zu erhalten, welche in Besitz ihrer privaten, geheimen Benutzerkennungen stehen.

Ein weiterer Vorteil des Verfahrens ist, dass selbst bei Wahl der gleichen Benutzerkennung durch verschiedene Benutzer aufgrund des Eingehens des Zufallswertes bei der Erzeugung des weiteren ersten Datenobjektschlüssel sichergestellt werden kann, dass niemals das gleiche Schlüsselpaar verschiedenen Benutzern zugeordnet wird.

Es sei hier darauf hingewiesen, dass das Verfahren auf beliebige Kryptosysteme zur Erzeugung asymmetrischer Schlüsselpaare angewendet werden können, wie zum Beispiel das RSA-, das Rabin- und das Elgamal-Kryptosystem oder kryptografische Verfahren auf elliptischen Kurven. Aus dem weiteren ersten Datenobjektschlüssel, den man basierend auf der Benutzerkennung und dem Zufallswert erhalten hat, wird der weitere zweite Datenobjektschlüssel berechnet, wobei für diese Berechnung solche Verfahren zur Anwendung kommen können.

Hierzu kann es erforderlich sein, dass der weitere erste Datenobjektschlüssel eine oder mehrere vorgegebene Eigenschaften haben muss und/oder Bedingungen erfüllen muss, die im Rahmen einer Zulässigkeitsprüfung überprüft werden. Wenn sich der weitere erste Schlüssel als für ein gewähltes Verfahren unzulässig erweist, so wird ein neuer Zufallswert generiert, um einen neuen Kandidaten für einen weiteren ersten Datenobjektschlüssel zu erzeugen, der dann wiederum einer Zulässigkeitsprüfung unterzogen wird. Dies wird solange wiederholt, bis ein zulässiger weiterer erster Datenobjektschlüssel gefunden worden ist. In diese Zulässigkeitsprüfung können Beschränkungen eingehen, welche sich direkt aus dem Algorithmus zur Durchführung eines entsprechenden asymmetrischen kryptografischen Schlüsselerzeugungsverfahrens ergeben.

Darüber hinaus können auch weitere Einschränkungen in die Zulässigkeitsprüfung eingehen, welche sich z.B. auf die Entropie des erzeugten Schlüssels beziehen oder aus aktuellen Erkenntnissen bezüglich der Angreifbarkeit des entsprechenden Schlüsselerzeugungsverfahrens ergeben. Beispielsweise gibt es für das RSA-Verfahren eine Reihe von allgemein bekannten und regelmäßig ergänzten Beschränkungen, deren Einhaltung bei einer Schlüsselerzeugung von Behördenstellen gefordert wird, um die Angreifbarkeit der erzeugten Schlüsselpaare zu minimieren. Beispielsweise spezifiziert PKCS#1 (Pub-lic Key Cryptography Standards) eine Reihe von kryptografischen Spezifikationen für RSA, welche von öffentlichen und privaten RSA-Schlüsselpaaren eingehalten werden müssen. Der in der Entwicklung befindliche Standard PKCS#13 wird die Anforderungen an die Schlüsselerzeugung auf elliptischen Kurven festsetzen.

Möglich ist, dass die Berechnung des weiteren ersten Datenobjektschlüssels unter Verwendung einer Funktion g, angewendet auf die Benutzerkennung pw erfolgt. Nach einer Ausführungsform wird entweder die beliebig wählbare Benutzerkennung als solche empfangen und daraufhin unter Verwendung der Funktion g umgewandelt, oder es wird direkt der Funktionswert g(pw) empfangen.

Die Berechnung des weiteren ersten Datenobjektschlüssels unter Verwendung des Wertes b = g(pw) und des Zufallswertes z hat den Vorteil, dass somit aus verhältnismäßig unsicheren Benutzerkennungen Eingangswerte berechnet werden können, welche eine hohe Zufälligkeit haben und somit in effektiver Weise bei der Berechnung des ersten Datenobjektschlüssels die Sicherheit desselben weiter erhöhen. Beispielsweise wird für g die kryptografische Hash-Funktion SHA-256 angewendet.

Möglich ist ferner, dass der weitere erste Datenobjektschlüssel durch Anwenden einer Funktion f auf die Werte b und z berechnet wird. Beispielsweise kann f als die Anwendung der kryptografischen Hash-Funktion SHA-256 auf die Konkatenation, also Hintereinanderhängung, von b und z definiert sein.

Durch Anwendung der Funktion f auf den Zufallswert z und den Funktionswert g(pw) wird eine hohe Qualität des weiteren ersten Datenobjektschlüssels gewährleistet. In anderen Worten, der weitere erste Datenobjektschlüssel weist aufgrund der zufälligen Wahl von z ebenfalls eine hohe Zufälligkeit auf, sodass ein Erraten des weiteren ersten Datenobjektschlüssels damit praktisch unmöglich gemacht wird.

Z.B. wird das Schlüsselpaar für ein Kryptosystem auf elliptischen Kurven berechnet. Eine elliptische Kurve ist gegeben durch die Gleichung y² = x³ + ax + b, wobei die Parameter a und b, wie auch die Koordinaten der Punkte (x,y) auf der Kurve ganze Zahlen aus dem Intervall [0, n-1] sind. Die Werte a, b, n, sowie ein gewählter Kurvenpunkt P bilden die sogenannten Domain-Parameter der elliptischen Kurve, welche zur Durchführung von kryptografischen Verfahren unter Verwendung des weiteren ersten und weiteren zweiten Schlüssels mit bekannt gegeben werden müssen. Die Anzahl der Punkte, die die Gleichung einer elliptischen Kurve erfüllen, bezeichnet man als die Ordnung der Kurve. Der erste Datenobjektschlüssel stellt eine natürliche Zahl dar, und der weitere zweite Datenobjektschlüssel, ein Punkt auf der Kurve, ist das Ergebnis der Multiplikation des weiteren ersten Datenobjektschlüssels mit dem Kurvenpunkt P der elliptischen Kurve.

Die Verwendung eines Kryptosystems auf elliptischen Kurven hat folgende Vorteile:
- Der erste Datenobjektschlüssel kann eine beliebige natürliche Zahl aus dem Intervall [1, n-1] sein. Sie ist an keine weiteren funktionalen Bedingungen gebunden; der Aspekt ihrer Beliebigkeit wird im weiteren Verlauf eine große Rolle spielen.
- Ein Kryptosystem auf elliptischen Kurven zu brechen hat eine sehr hohe Komplexität, die viel höher ist als bei RSA.
- Die Schlüssel sind im Vergleich zu RSA sehr kurz und die Berechnungen auf der Kurve relativ einfach, womit sie vielseitig und effizient implementiert werden können.
- Der weitere zweite Datenobjektschlüssel kann aus dem weiteren ersten Datenobjektschlüssel einfach und jederzeit wieder berechnet werden.

Über die Funktionen f und g kann sehr effizient aus der Benutzerkennung und dem Zufallswert der weitere erste Datenobjektschlüssel berechnet werden. Damit ist es möglich, durch mathematische Funktionen der gewählten Benutzerkennung das kryptografische Schlüsselpaar zuzuordnen. Wegen dieses funktionellen Zusammenhangs ist hier ein Vorhalten einer tabellarischen Zuordnung von Schlüsselpaar und einer entsprechenden Benutzerkennung nicht notwendig.

Möglich ist ferner die Durchführung des Schrittes der Zulässigkeitsprüfung des ersten Datenobjektschlüssels. Im Rahmen der Zulässigkeitsprüfung wird geprüft, ob der weitere erste Datenobjektschlüssel größer als 1 und kleiner als die Ordnung der elliptischen Kurve ist. Bei Erfüllung dieser Prüfbedingung sind der Zufallswert sowie der weitere erste und der weitere zweite Datenobjektschlüssel zulässig. Ist die Prüfbedingung jedoch nicht erfüllt, wird ein neuer Zufallswert gewählt, mit dem ein erneutes Berechnen des weiteren ersten Datenobjektschlüssels erfolgt sowie ein erneutes Durchführen der Zulässigkeitsprüfung dieses Datenobjektschlüssels. Dieser Vorgang wird wiederholt, bis die Zulässigkeitsprüfung bestanden wird.

Die Zulässigkeitsprüfung kann um weitere Prüfbedingungen erweitert werden, z.B. um die Prüfung, dass der weitere erste Datenobjektschlüssel eine hohe Zufälligkeit aufweist. Hierzu sei angemerkt, dass in der Kryptografie üblicherweise mit algebraischen Strukturen gearbeitet wird, die nur endlich viele Elemente enthalten. Dies hat seine Ursache darin, dass im Falle einer endlichen Anzahl von Elementen viele in den reellen Zahlen harmlose Probleme schwierig werden, sodass elliptische Kurven mit endlich vielen Elementen effektiv für kryptografische Anwendungen verwendet werden können. Für kryptografische Anwendungen ist es nun wichtig, dass die verwendete algebraische Struktur groß genug ist, das heißt, dass die Anzahl der Punkte auf einer elliptischen Kurve, als Ordnung bezeichnet, ausreichend groß ist. In diesem Kontext muss man in Betracht ziehen, dass der erzeugte weitere erste Datenobjektschlüssel größer sein kann als die Ordnung der elliptischen Kurve. Um hier dennoch eine Assoziation zu ermöglichen, ist es üblich, eine Division des weiteren ersten Datenobjektschlüssels modulo der Ordnung der elliptischen Kurve durchzuführen. Damit ergibt sich jedoch eine hohe Wahrscheinlichkeit, dass die resultierende Zahl sich in einem unteren Wertebereich des Intervalls [2, r-1] (mit r als der Ordnung der elliptischen Kurve) befindet oder sogar 0 oder 1 ist, sodass sich hiermit die Schwierigkeit reduziert, einen in diesem Wertebereich liegenden Punkt der Kurve mathematisch oder durch Ausprobieren herauszufinden. Durch die Durchführung der Zulässigkeitsprüfung wird somit eine Einschränkung des Wertebereichs, in welchem sich der weitere erste Datenobjektschlüssel befindet, zuverlässig vermieden, sodass sich hiermit die Entropie des weiteren ersten Datenobjektschlüssels und damit dessen Zufälligkeit in ausreichendem Maße sicherstellen lässt.

Ein weiterer Vorteil der Zulässigkeitsprüfung ist, dass hiermit sichergestellt werden kann, dass eine Verträglichkeit des weiteren ersten Datenobjektschlüssels mit entsprechenden Programmbibliotheken für elliptische Kurven, wie sie nach dem Stand der Technik verfügbar sind, zuverlässig gewährleistet werden kann.

Es sei hier darauf hingewiesen, dass zur Durchführung des Verfahrens zur Erzeugung eines asymmetrischen kryptografischen Schlüsselpaares unter Verwendung einer elliptischen Kurvenfunktion die Durchführung der Zulässigkeitsprüfung nicht zwingend notwendig ist. Auch ohne Anwendung der Zulässigkeitsprüfung lassen sich hier Schlüsselpaare erzeugen, welche jedoch unter Umständen je nach Benutzerkennung und Zufallswert nicht sehr hohen Sicherheitsanforderungen Rechnung tragen können, welche für kryptografische Anwendungen gefordert sein könnten. Die Zulässigkeitsprüfung ist im Falle elliptischer Kurven ein weiterer Schritt, um sicherzustellen, dass die erzeugten Schlüsselpaare eben jenen Sicherheitsanforderungen genügen.

Z.B. ist die Bitlänge des Zufallswertes größer oder gleich der Bitlänge der Ordnung der elliptischen Kurve. Außerdem ist nach einer Ausführungsform der Erfindung der Zufallswert so gewählt, dass der Wert des erzeugten weiteren ersten Datenobjektschlüssels kleiner als die Ordnung der elliptischen Kurve ist. Beide Kriterien haben ebenfalls, wie bereits für die Zulässigkeitsprüfung diskutiert, denselben Effekt, nämlich dass somit eine hohe Entropie des weiteren ersten Datenobjektschlüssels gewährleistet werden kann. Damit wird in anderen Worten die Sicherheit des weiteren ersten Datenobjektschlüssels und damit die Sicherheit des Verschlüsselungsverfahrens signifikant erhöht.

Z.B. wird das Schlüsselpaar für ein RSA-Kryptosystem berechnet. Ein RSA-Kryptosystem ist gegeben durch eine Zahl n, die das Produkt zweier Primzahlen p und q ist (n = p · q), der Zahl d, die der Bedingung ggT(d, (p - 1)·(q - 1)) = 1 genügt und der Zahl e, die der Bedingung e·d ≡ 1 mod (p - 1)·(q - 1) genügt ("ggT" steht für größter gemeinsamer Teiler). Nach der Wahl von d und der Berechnung von e müssen die Werte p, q und (p - 1)·(q - 1) gelöscht werden. Welche der beiden Zahlen e und d der öffentliche und welche der private Schlüssel ist, ist bei RSA grundsätzlich frei wählbar; in dieser Erfindung berechnen die Funktionen f und g aus der Benutzerkennung pw und dem Zufallswert z den weiteren ersten Datenobjektschlüssel d. Über den erweiterten euklidschen Algorithmus wird dann aus dem weiteren ersten Datenobjektschlüssel d der weitere zweite Datenobjektschlüssel e berechnet.

Die Vorteile des RSA-Verfahrens sind die Tatsachen, dass das Verfahren bei entsprechend lang gewählten Schlüsseln nach wie vor sehr sicher ist und dass es eine hohe Verbreitung besitzt. Jedoch besitzt RSA auch die Nachteile, dass es aufgrund der erforderlichen großen Schlüssellänge im Betrieb langsam ist und moderne Faktorisierungsalgorithmen Anlass zu der Befürchtung geben, dass RSA in nicht allzu ferner Zukunft gebrochen wird.

Auch für RSA ist es über die Funktionen f und g möglich, aus der Benutzerkennung und dem Zufallswert den weiteren ersten Datenobjektschlüssel zu berechnen. Damit ist es auch für RSA möglich, durch mathematische Funktionen der gewählten Benutzerkennung ein kryptografisches Schlüsselpaar zuzuordnen. Wegen dieses funktionellen Zusammenhangs ist ein Vorhalten einer tabellarischen Zuordnung von Schlüsselpaar und einer entsprechenden Benutzerkennung auch für RSA nicht notwendig.

Möglich ist auch ein Schritt der Zulässigkeitsprüfung des ersten RSA-Datenobjektschlüssels. Im Rahmen der Zulässigkeitsprüfung wird geprüft, ob der weitere erste Datenobjektschlüssel d die Bedingungen
- d liegt im Intervall [2, (p - 1)·(q - 1) - 2] und
- ggT(d, (p - 1 )·(q - 1 )) = 1
erfüllt. Bei Erfüllung dieser Prüfbedingungen sind der Zufallswert sowie der erste und der weitere zweite Datenobjektschlüssel zulässig. Ist die Prüfbedingung jedoch nicht erfüllt, wird ein neuer Zufallswert z gewählt, mit dem ein erneutes Berechnen des weiteren ersten Datenobjektschlüssels erfolgt sowie ein erneutes Durchführen der Zulässigkeitsprüfung dieses Datenobjektschlüssels. Dieser Vorgang wird wiederholt, bis die Zulässigkeitsprüfung bestanden wird.

Z.B. wird der Zufallswert von einer Datenbank abgerufen, wobei der Zufallswert eindeutig der Benutzeridentifikation zugeordnet ist. Beispielsweise wird bei einer ersten Durchführung des Verfahrens zur Erzeugung des asymmetrischen Schlüsselpaares einmalig ein Zufallswert von einer vertrauenswürdigen Stelle, z.B. einer Zertifizierungsstelle erzeugt, welcher im Falle der Zulässigkeit des weiteren ersten Datenobjektschlüssels für kryptografische Vorgänge einem entsprechenden Benutzer indirekt zugänglich sein muss. Durch Abspeichern des Zufallswerts in einer Datenbank, zugeordnet der eindeutigen Benutzeridentifikation, kann ein Computerprogramm, welches das Verfahren zur Erzeugung von asymmetrischen Schlüsselpaaren ausführt, den Zufallswert über eine sichere Kommunikationsverbindung anhand der Benutzeridentifikation abrufen und dazu verwenden, den entsprechenden ersten und gegebenenfalls auch den weiteren zweiten Datenobjektschlüssel zu generieren.

Vorzugsweise ist der Zufallswert verschlüsselt in der Datenbank abgelegt. Hierzu kann nach einer Ausführungsform der Erfindung eine symmetrische Verschlüsselung, z.B. unter Verwendung von AES-256, Anwendung finden. Die Verwendung eines verschlüsselten Zufallswertes hat den Vorteil, dass damit Wörterbuchangriffe zur versuchsweisen Entschlüsselung des ersten Schlüssels verhindert werden können.

Z.B. sind die Computerprogrammprodukte durch Applets oder Browser Plugins ausgebildet. Ebenso ist es möglich, die Computerprogrammprodukte als eigenständige Anwendungen für ein Datenverarbeitungssystem bereitzustellen. Die Verwendung eines Applets oder eines Browser Plugins hat den Vorteil, dass bestehende Datenverarbeitungssysteme zur Durchführung des Verfahrens zur Schlüsselerzeugung und konsequenter Weise auch zur Durchführung von kryptografischen Operationen wie Verschlüsselung, Entschlüsselung sowie Erstellung und Verifikation von digitalen Signaturen, nicht umgerüstet werden müssen: Hier genügt lediglich das Laden eines Applets, beispielsweise über das Internet, welches in sicherer Weise die beschriebenen Operationen durchführen kann.

Z.B. wird das weitere asymmetrische kryptografische Schlüsselpaar von einem tragbaren Datenträger empfangen, wobei das Chiffrat auf dem tragbaren Datenträger gespeichert wird. Beispielsweise handelt es sich bei dem tragbaren Datenträger um eine Chipkarte, ein Ausweisdokument oder um ein mobiles Telekommunikationsgerät wie ein Mobiltelefon oder ein Personal Digital Assistant (PDA).

In einem weiteren Aspekt betrifft die Erfindung ein Computerprogrammprodukt mit von einem Prozessor ausführbaren Instruktionen zur Durchführung der obig beschriebenen Verfahrensschritte.

Im Folgenden werden Ausführungsformen der Erfindung anhand von Zeichnungen näher erläutert. Es zeigen:
- Figur 1:: ein Blockdiagramm eines Datenverarbeitungssystems,
- Figur 2:: ein Flussdiagramm eines Verfahrens zur Bereitstellung von Berechtigungsschlüsseln und zur Verschlüsselung von Daten,
- Figur 3:: ein Flussdiagramm eines Verfahrens zur Entschlüsselung von Daten,
- Figur 4:: ein Flussdiagramm eines Verfahrens zur asymmetrischen Verschlüsselung von Daten,
- Figur 5:: ein Flussdiagramm eines Verfahrens zur Entschlüsselung von Daten mittels eines asymmetrischen Schlüsselverfahrens,
- Figur 6:: ein Flussdiagramm eines Verfahrens zur Berechnung von asymmetrischen Schlüsseln nach dem RSA-Verfahren,
- Figur 7:: ein weiteres Flussdiagramm eines Verfahrens zur hierarchischen Bereitstellung von Berechtigungsschlüsseln und zur Verschlüsselung von Daten,
- Figur 8:: ein weiteres Flussdiagramm zum Entschlüsseln von Datenobjekten.

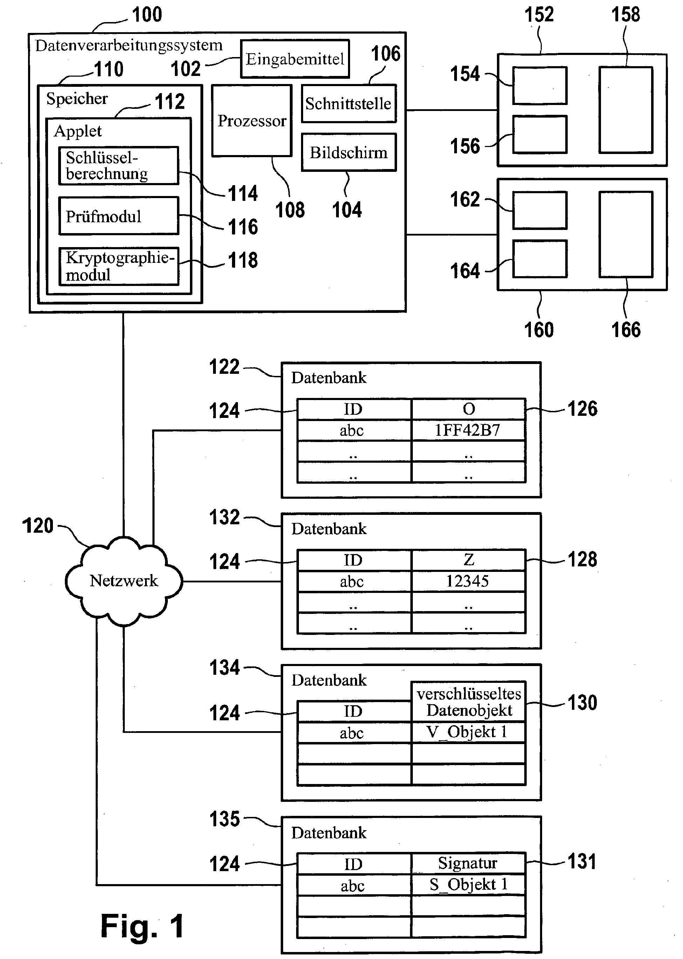

Die Figur 1 zeigt ein Datenverarbeitungssystem 100. Das Datenverarbeitungssystem umfasst Eingabemittel 102, wie zum Beispiel eine Tastatur, eine Maus, ein Pinpad, Mittel zur Erfassung von biometrischen Merkmalen, wie zum Beispiel einen Fingerabdruckscanner oder einen Irisscanner. Ferner umfasst das Datenverarbeitungssystem 100 einen Bildschirm 104 sowie eine Schnittstelle 106, welche zum Beispiel zur Kommunikation mit einem Netzwerk 120, wie dem Internet, verwendet werden kann. Ferner umfasst das Datenverarbeitungssystem 100 einen Prozessor 108, welcher dazu ausgebildet ist, ausführbare Instruktionen zur Durchführung von Verfahrensschritten auszuführen. Diese Instruktionen sind beispielsweise in Form eines Applets 112 im Speicher 110 enthalten.

Beispielsweise kann das Datenverarbeitungssystem 100 zur Erzeugung von asymmetrisch kryptografischen Schlüsselpaaren und zur anschließenden Verschlüsselung und Entschlüsselung von Datenobjekten sowie zur Erzeugung und Verifikation von digitalen Signaturen und für weitere kryptografische Operationen verwendet werden. Dies erfordert zunächst eine Berechnung von Schlüsselpaaren, was beispielsweise mittels des Moduls 114 des Applets 112 erfolgen kann. Zur Berechnung von Schlüsseln wird hierbei mittels des Moduls 114 wie folgt vorgegangen: Über die Eingabemittel 102 wird zunächst eine beliebig wählbare Benutzerkennung von einem Benutzer erhalten. Aus der Benutzerkennung wird daraufhin ein erster Datenobjektschlüssel berechnet, wobei in die Berechnung ein Zufallswert, welcher durch das Datenverarbeitungssystem 100 erzeugt wird, und die Benutzerkennung eingehen. Bei dem hier berechneten ersten Datenobjektschlüssel handelt es sich um einen privaten Schlüssel des Benutzers, wobei es möglich ist, dass zur Verwendung des ersten Datenobjektschlüssels in kryptografischen Anwendungen zusätzliche Parameter mit veröffentlicht werden müssen, um den ersten Datenobjektschlüssels für die Durchführung kryptografischer Operationen zu nutzen. Wie bereits oben angemerkt, ist es im Falle von elliptischen Kurven notwendig, zusätzlich zum ersten und zweiten Datenobjektschlüssel auch die Domainparameter der elliptischen Kurve zur Verfügung zu stellen, welche in Kombination mit dem ersten und zweiten Datenobjektschlüssel die Anwendung kryptografischer Operationen möglich macht. Ähnlich gilt für RSA, dass die natürliche Zahl n mit veröffentlicht werden muss, um kryptografische Operationen durchführen zu können.

Nach Berechnung des ersten Datenobjektschlüssels erfolgt eine Prüfung des Datenobjektschlüssels mittels des Prüfmoduls 116. Diese Prüfung dient einer Zulässigkeitsprüfung des ersten Datenobjektschlüssels, nämlich, ob der erzeugte erste Datenobjektschlüssel verschiedenen Sicherheitsaspekten genügt.

Beispielsweise wird bei elliptischen Kurven der öffentliche Schlüssel, d.h. der zweite Datenobjektschlüssel, aus dem ersten privaten Datenobjektschlüssel berechnet, indem ein Kurvenpunkt einer elliptischen Kurve mit dem geheimen Schlüssel multipliziert wird. In diesem Fall besteht die Zulässigkeitsprüfung des ersten Datenobjektschlüssels darin, zu prüfen, ob der erste Datenobjektschlüssel größer als 1 und kleiner als die Ordnung der elliptischen Kurve ist, wobei bei Erfüllung dieser Prüfbedingung der Zufallswert und der erste und zweite Datenobjektschlüssel zulässig sind. Ist dies jedoch nicht der Fall, muss ein neuer erster Datenobjektschlüssel und folglich auch ein neuer zweiter Datenobjektschlüssel berechnet werden, indem ein neuer Zufallswert gewählt wird und das Verfahren zur Schlüsselberechnung mittels des Moduls 114 sowie das Verfahren zur Prüfung der erzeugten Schlüssel mittels des Moduls 116 wiederholt durchgeführt wird.

Der zur Schlüsselberechnung verwendete Zufallswert wird daraufhin in einer Datenbank 132 abgelegt und gegebenenfalls verschlüsselt. Dies erfolgt beispielsweise so, dass für den entsprechenden Benutzer eine eindeutige Benutzeridentifikation vergeben wird, wobei dieser Benutzeridentifikation 124 in einer Tabelle der Datenbank 132 der zuvor erzeugte Zufallswert 128 zugeordnet wird. Im vorliegenden Beispiel der Figur 1 ist der Benutzerkennung "abc" der Zufallswert Z mit dem Wert "12345" zugeordnet. Wie bereits oben erwähnt, wird hier vorzugsweise der Zufallswert in verschlüsselter Form in der Datenbank 132 gespeichert, um zuverlässig Wörterbuchangriffe auf den ersten Datenobjektschlüssel zu verhindern.

In einer weiteren Datenbank 122 ist ebenfalls der Benutzeridentifikation 124 der mittels des Schlüsselberechnungsmoduls 114 erzeugte öffentliche Schlüssel 126 zugeordnet abgespeichert. Beispielsweise ist wiederum der Benutzeridentifikation "abc" der öffentliche Schlüssel "1FF42B7" zugeordnet.

Im Folgenden sei angenommen, dass in einer Datenbank 134 ebenfalls der Benutzeridentifikation 124 zugeordnet ein Datenobjekt 130 verschlüsselt gespeichert ist. Das Datenobjekt ist dabei mit dem öffentlichen Schlüssel 126 verschlüsselt, welcher in der Datenbank 122 abgelegt ist. Zur Entschlüsselung des Datenobjekts 130 wird nun wie folgt vorgegangen: Über die Eingabemittel 102 gibt ein Benutzer seine Benutzeridentifikation und die in der Benutzeridentifikation gewählte Benutzerkennung ein. Daraufhin erfolgt mittels des Moduls 114 die Berechnung des ersten Datenobjektschlüssels unter Verwendung des Zufallswertes 128, welcher anhand der Benutzeridentifikation 124 von der Datenbank 132 abgerufen wird. In diese Berechnung des ersten Datenobjektschlüssels gehen, wie bereits oben erwähnt, der Zufallswert 128 und die Benutzerkennung ein, welche zuvor über die Eingabemittel 102 in das Datenverarbeitungssystem eingegeben wurde.

Mittels des nun so erzeugten geheimen und privaten Datenobjektschlüssels ist es nun möglich, das Datenobjekt 130 zu entschlüsseln.

In einer Datenbank 135 kann zumindest eine Signatur 131 S_Objekt 1 des Datenobjekts sowie optional das Datenobjekt 130 selbst gespeichert sein. Das Datenobjekt 130 ist dabei mit dem geheimen Schlüssel signiert, welcher dem öffentlichen Schlüssel 126 zugeordnet ist. Die Verifikation der Signatur erfolgt dementsprechend mit dem öffentlichen Schlüssel 126.

An dieser Stelle sei angemerkt, dass die beliebig wählbare Benutzerkennung, welche über die Eingabemittel 102 in das Datenverarbeitungssystem 100 eingegeben wird, beispielsweise eine Zahlenkombination, eine Zahlen-Buchstaben-Kombination oder auch ein biometrisches Merkmal sein kann. Beispielsweise kann im Falle der Verwendung eines biometrischen Merkmals aus den biometrischen Daten eine Bitfolge in eindeutiger Weise berechnet werden, welche daraufhin als Benutzerkennung in die Schlüsselberechnung mittels des Moduls 114 eingehen kann.

Ferner sei angemerkt, dass insbesondere bei der Ver- und Entschlüsselung von medizinischen Datenobjekten durch das Datenverarbeitungssystem 100 beispielsweise wie folgt vorgegangen wird: Über die Schnittstelle 106 wird beispielsweise ein medizinisches Datenobjekt von einem bildgebenden medizinischen Instrument wie einem Röntgengerät empfangen. Röntgendaten sind typischerweise Bilddaten, weiche umfangreiche Datenmengen darstellen. Das Datenverarbeitungssystem erzeugt einen zufälligen symmetrischen Schlüssel, mit welchem diese medizinischen Röntgendaten verschlüsselt werden. Daraufhin werden diese verschlüsselten Daten auf der Datenbank 134 mit der eindeutigen Benutzeridentifikation 124 assoziiert abgelegt. Daraufhin erfolgt eine Verschlüsselung des erzeugten symmetrischen Schlüssels mit dem öffentlichen Schlüssel 126. Dieser so verschlüsselte symmetrische Schlüssel wird ebenfalls auf der Datenbank 134 mit der Benutzeridentifikation 124 und den verschlüsselten Daten assoziiert abgelegt.

Zur Entschlüsselung wird nun der verschlüsselte symmetrische Schlüssel mittels des Kryptografiemoduls 118 entschlüsselt, indem der entsprechende private Schlüssel unter Verwendung der Benutzerkennung wie oben beschrieben erzeugt und für die Entschlüsselung verwendet wird. Mit dem so erhaltenen symmetrischen Schlüssel ist es daraufhin möglich, das verschlüsselte Datenobjekt 130 zu entschlüsseln.

Vorzugsweise werden Datenobjekte 130 in der Datenbank 134 jeweils einzeln verschlüsselt abgespeichert. Selbst im Falle eines Satzes von semantisch zusammengehörenden Datenobjekten wird vorzugsweise jedes einzelne Datenobjekt für sich verschlüsselt in der Datenbank 134 abgespeichert, so dass im Falle eines Abrufens eines einzelnen Datenobjekts eine Übertragung dieses verschlüsselten Datenobjekts 130 an das Datenverarbeitungssystem 100 erfolgt, woraufhin dort seine Entschlüsselung vorgenommen wird. Würde man im Gegensatz dazu für semantisch zusammengehörende Datenobjekte, die in einem einzigen Datenobjekt zusammengefasst, verschlüsselt und abgespeichert wären, aus Gründen der Minimierung des zu transportierenden Datenvolumens die Entschlüsselung in der Datenbank vornehmen, so hätte der Betreiber Zugriff auf die entschlüsselten Datenobjekte. Dahingegen hat die oben beschriebene Vorgehensweise den Vorteil, dass zu keinem Zeitpunkt die Datenbank 134, bzw. deren Betreiber Zugriff auf entschlüsselte Schlüssel oder Datenobjekte erhält.

Das Datenverarbeitungssystem 100 ist ferner dazu ausgebildet, um Berechtigungsschlüssel in hierarchischer Weise bereitzustellen und diese zur Ver- und Entschlüsselungsvorgängen von Datenobjekten zu verwenden. Im Folgenden sei ohne Beschränkung der Allgemeinheit angenommen, dass beispielsweise ein Benutzer im Besitz einer Chipkarte 152 ist, auf welcher ein privater Berechtigungsschlüssel 154 und ein öffentlicher Berechtigungsschlüssei 156 abgespeichert ist. Soll nun ein neues Datenobjekt verschlüsselt werden, kann hierfür das obig beschriebene Verfahren unter Verwendung eines zusätzlichen symmetrischen Schlüssels zum Einsatz kommen, mit welchem ein Datenobjekt verschlüsselt wird. Das so mit dem symmetrischen Schlüssel verschlüsselte Datenobjekt 130 wird zusammen mit der eindeutigen Benutzeridentifikation 124 assoziiert in der Datenbank 134 abgelegt.

Abweichend von obiger Beschreibung erfolgt jedoch nun eine Verschlüsselung des symmetrischen Schlüssels mit einem initialen öffentlichen Schlüssel, welcher beispielsweise in der Datenbank 122 als initialer öffentlicher Schlüssel 126 abgelegt ist. Außerdem ist beispielsweise ebenfalls auf der Datenbank 122 ein Chiffrat 150 abgelegt. Dieses Chiffrat 150 ist dabei eindeutig der Chipkarte 152 zuordenbar.

Findet nun ein solcher Verschlüsselungsvorgang des obig erwähnten symmetrischen Schlüssels statt, wird zunächst der eindeutigen Benutzeridentifikation 124 zugeordnete initiale Berechtigungsschlüssel in Form eines öffentlichen Schlüssels 126 von der Datenbank 122 abgerufen. Mittels des Kryptografiemoduls 118 erfolgt daraufhin eine Verschlüsselung des symmetrischen Schlüssels mit dem initialen öffentlichen Berechtigungsschlüssel. Dieser verschlüsselte symmetrische Schlüssel wird daraufhin in der Datenbank 134 mit dem verschlüsselten Datenobjekt 130 assoziiert abgelegt.

Für einen Entschlüsselungsvorgang des verschlüsselten Datenobjektes 130 wird eine Entschlüsselung des symmetrischen Datenobjektschlüssels benötigt. Dies erfordert die Kenntnis des privaten initialen Berechtigungsschlüssels, welche zusammen mit dem initialen öffentlichen Berechtigungsschlüssel ein asymmetrisches kryptografisches Schlüsselpaar bildet. Zum Erhalt des privaten initialen Berechtigungsschlüssels dient nun das Chiffrat 150, welches der Chipkarte 152 zugeordnet ist. Das Chiffrat enthält in verschlüsselter Weise den initialen privaten Berechtigungsschlüssel, welcher mit dem öffentlichen Berechtigungsschlüssel 156 der Chipkarte 152 zuvor verschlüsselt wurde. Unter Verwendung der Schnittstelle 106 ist nun das Kryptografiemodul 118 in der Lage, aus dem Chiffrat 150 unter Verwendung des privaten Berechtigungsschlüssels 154 der Chipkarte 152 den initialen privaten Berechtigungsschlüssel zu extrahieren. Mittels dieses initialen privaten Berechtigungsschlüssels ist daraufhin das Kryptografiemodul 118 in der Lage, den verschlüsselten symmetrischen Schlüssel, welcher mit dem verschlüsselten Datenobjekt 130 assoziiert ist, zu entschlüsseln, um daraufhin mit dem so erhaltenen symmetrischen Schlüssel das verschlüsselte Datenobjekt 130 selbst zu entschlüsseln.

Nun sei angenommen, dass ein Benutzer der Chipkarte 152 diese Chipkarte durch eine neue Chipkarte 160 ersetzen möchte, bzw. neben der Chipkarte 152 eine zusätzliche Chipkarte 160 zur Durchführung von Ver- und Entschlüsselungsvorgängen von Datenobjekten einsetzen möchte. Dies erfordert eine hierarchische Bereitstellung von Berechtigungsschlüsseln, was wie folgt geschieht:
Es sei angenommen, dass sich auf der Chipkarte 160 bereits ein neuer privater Berechtigungsschlüssel 162 und ein neuer öffentlicher Berechtigungsschlüssel 164 befinden. Angemerkt sei hier, dass sich dieses Vorhandensein von Berechtigungsschlüsseln 162 und 164 auf einer Chipkarte 160 ohne Weiteres durch das obig beschriebene Verfahren zur Erzeugung von Datenobjektschlüsseln ersetzen lässt, welche mittels einer beliebig wählbaren Benutzerkennung in das Datenverarbeitungssystem 100 eingegeben werden. Im letzteren Fall würde die Chipkarte 160 wegfallen - ein Bereitstellen von Datenobjektschlüsseln würde unter Verwendung der besagten Benutzerkennung durch das Datenverarbeitungssystem 100 selbst erfolgen. Im Folgenden sei jedoch angenommen, dass ohne Beschränkung der Allgemeinheit ein Benutzer zusätzlich die Chipkarte 160 mit den Berechtigungsschlüsseln 162 und 164 zum Einsatz bringen möchte. Hierzu werden entweder in einer vom Datenverarbeitungssystem mittels des Bildschirms angegebenen Reihenfolge die Chipkarten 152 und 160 in ein entsprechendes Lesegerät des Datenverarbeitungssystems 100 eingeführt, oder aber das Datenverarbeitungssystem 100 ist in der Lage, gleichzeitig beide Chipkarten 152 und 160 zu lesen. Im Folgenden sei angenommen, dass ein gleichzeitiger Zugriff auf die Chipkarten 152 und 160 möglich ist.

Nachdem ein Benutzer die beiden Chipkarten 152 und 160 in entsprechende Lesegeräte des Datenverarbeitungssystems 100 eingeführt hat, liest das Datenverarbeitungssystem 100 das Chiffrat 150 aus der Datenbank 122 aus, welches der "alten Chipkarte" 152 zugeordnet war. Wie bereits oben im Detail beschrieben, ist damit das Datenverarbeitungssystem 100 in der Lage, den initialen privaten Berechtigungsschlüssel aus dem Chiffrat unter Verwendung des privaten Berechtigungsschlüssels 154 zu extrahieren. Der so gewonnene initiale private Berechtigungsschlüssel wird daraufhin mit dem öffentlichen Berechtigungsschlüssel 164 der neuen Chipkarte 160 verschlüsselt. Dieser verschlüsselte initiale Berechtigungsschlüssel bildet wiederum ein neues Chiffrat 150, welches mit der Chipkarte 160 assoziiert in der Datenbank 122 abgelegt wird. Die Chiffratberechnung erfolgt dabei mit dem Modul 114.

Eine Verschlüsselung von Daten erfolgt nun wie obig im Detail beschrieben unter Verwendung des symmetrischen Schlüssels, welcher grundsätzlich, unabhängig vom Einsatz der Chipkarte 152 oder 160 mit dem initialen öffentlichen Schlüssel 126 verschlüsselt und mit dem entsprechenden verschlüsselten Datenobjekt 130 assoziiert in der Datenbank 134 abgelegt wird. Erfolgt nun in umgekehrter Reihenfolge ein Entschlüsselungsvorgang eines verschlüsselten Datenobjektes 130, so kann dies entweder unter Verwendung der Chipkarte 152 oder der Chipkarte 160 erfolgen. In beiden Fällen wird jeweils der Chipkarte zugeordnete private Schlüssel 154 bzw. 162 dazu verwendet, um das der jeweilig verwendeten Chipkarte zugeordnete Chiffrat zu entschlüsseln. Der so erhaltene initiale private Schlüssel kann nun wiederum dazu verwendet werden, um einen Entschlüsselungsvorgang des symmetrischen Datenobjektschlüssels vorzunehmen, welcher mit dem verschlüsselten Datenobjekt 130 in der Datenbank 134 assoziiert abgelegt ist.

Es sei hier darauf hingewiesen, dass vorzugsweise die privaten Schlüssel 154 und 162 nie die Chipkarten 152 und 160 verlassen. Aus diesem Grund weisen die Chipkarten 152 und 160 entsprechende Hardware- oder Softwaremodule 158 bzw. 166 auf, mit welchen entsprechende Kryptografievorgänge, wie beispielsweise Entschlüsselungsvorgänge des Chiffrats 150 vorgenommen werden können. Ebenfalls sollte vorzugsweise eine Verschlüsselung des initialen privaten Schlüssels in den Modulen 158 bzw. 166 selbst erfolgen, sodass der durch die Chipkarten 152 und 160 extrahierte initiale erste Berechtigungsschlüssel nie diese Chipkarten verlässt, sodass ein Missbrauch des initialen privaten Berechtigungsschlüssels ausgeschlossen ist.

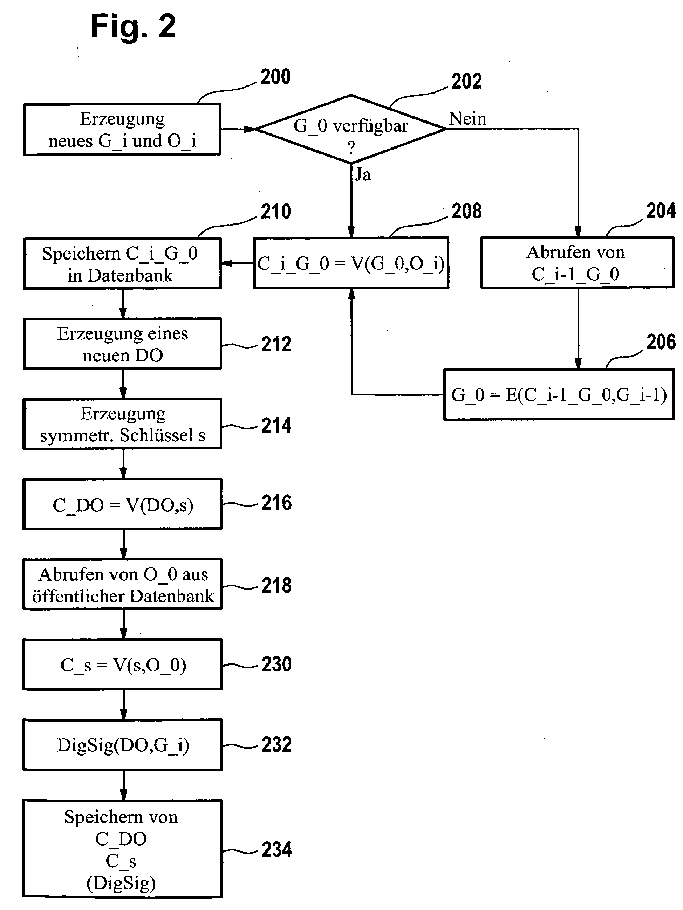

Die Figur 2 zeigt ein Verfahren zur Durchführung von Verfahrensschritten zur Bereitstellung von Berechtigungsschlüsseln als auch zur Verschlüsselung eines Datenobjekts. Das Verfahren beginnt im Schritt 200, in welchem ein neuer privater Schlüssel Gᵢ und öffentlicher Schlüssel Oᵢ erzeugt wird. Dies kann beispielsweise durch Wahl einer neuen Benutzerkennung wie obig beschrieben oder durch Bereitstellung einer neuen Chipkarte erfolgen, in welcher bereits ein neuer privater Berechtigungsschlüssel Gᵢ und ein neuer öffentlicher Berechtigungsschlüssel Oᵢ eingespeichert ist. In Schritt 202 wird überprüft, ob ein initialer privater Berechtigungsschlüssel mit Index i = 0 verfügbar ist. Typischerweise wird dies lediglich dann der Fall sein, wenn bisher außer dem initialen privaten Berechtigungsschlüssel und dem zugehörigen initialen öffentlichen Berechtigungsschlüssel keine weiteren asymmetrischen kryptographischen Schlüsselpaare erzeugt wurden. Allerdings sei im Folgenden auch hier wieder ohne Beschränkung der Allgemeinheit angenommen, dass bereits ein Satz von verschiedenen asymmetrischen Schlüsselpaaren existiert, welche Teil einer Schlüsselpaarsequenz sind. Dementsprechend existieren zum Zeitpunkt der Erzeugung des privaten Schlüssels Gᵢ und des öffentlichen Schlüssels Oᵢ insgesamt außerdem dem initialen Schlüsselpaar i-1 weitere asymmetrische kryptografische Schlüsselpaare.

Es sei angenommen, dass der initiale private Berechtigungsschlüssel mit Index 0 G₀ nicht verfügbar ist, sodass nach Schritt 202 der Schritt 204 erfolgt, in welchem ein Chiffrat, welches der Chipkarte mit Index i-1 zugeordnet ist, von einer Datenbank abgerufen wird. Alternativ ist es auch möglich, dass dieses Chiffrat auf der Chipkarte, welches dem Index i-1 zugeordnet ist, abgespeichert ist.

Daraufhin wird in Schritt 206 unter Verwendung des privaten Berechtigungsschlüssels der Chipkarte mit Index i-1 das Chiffrat entschlüsselt. Durch Entschlüsseln des Chiffrats erhält man in Schritt 206 den initialen privaten Berechtigungsschlüssel. Dieser initiale private Berechtigungsschlüssel G₀ wird daraufhin in Schritt 208 erneut verschlüsselt, wobei hier eine Verschlüsselung unter Verwendung des neuen öffentlichen Schlüssels Oᵢ stattfindet. Das so erhaltene neue Chiffrat wird in Schritt 210 der neuen Chipkarte zugeordnet in einer Datenbank abgespeichert.

Der direkte Übergang von Schritt 202 nach Schritt 208 ist üblicherweise nur dann notwendig, wenn die neue Chipkarte mit den Schlüsseln Gᵢ und Oᵢ die Chipkarte mit Index i=1 ist.

Nachdem in den Schritten 200 bis 210 eine hierarchische Bereitstellung von Berechtigungsschlüsseln erfolgt ist, folgt in den Schritten 212 bis 234 ein Verschlüsselungsvorgang von Datenobjekten. In anderen Worten, müssen die Schritte 200 bis 210 lediglich einmalig bei Ausgabe einer neuen Chipkarte mit neuen asymmetrischen Schlüsseln durchgeführt werden, wohingegen die Schritte 212 bis 234 für jeden Verschlüsselungsvorgang von Datenobjekten durchgeführt werden müssen.

In Schritt 212 wird nun ein neues Datenobjekt erzeugt. Daraufhin erfolgt in Schritt 214 die Erzeugung eines symmetrischen Schlüssels s, welcher in Schritt 216 dazu verwendet wird, um das Datenobjekt des Schritts 212 zu verschlüsseln. In Schritt 218 erfolgt das Abrufen des initialen öffentlichen Berechtigungsschlüssels O₀ aus einer öffentlichen Datenbank, wobei in Schritt 230 dieser initiale öffentliche Berechtigungsschlüssel zur Verschlüsselung des symmetrischen Schlüssels, welcher in Schritt 214 erhalten wurde, verwendet wird.

Schließlich erfolgt eine Speicherung des verschlüsselten Datenobjektes und des verschlüsselten symmetrischen Schlüssels in einer entsprechenden Patientendatenbank in Schritt 234.

Der Schritt 232, welcher nach Schritt 230 folgt, ist ein optionaler Schritt, in welchem die Möglichkeit besteht, das Datenobjekt zusätzlich zu signieren, um so einem unbefugten Missbrauch und einer Manipulation des Datenobjektes vorzubeugen. Eine solche digitale Signierung erfolgt vorzugsweise mit dem privaten Berechtigungsschlüssel Gᵢ der aktuell verwendeten Chipkarte. Wurde der Schritt 232 durchgeführt, wird die digitale Signatur zusätzlich in Schritt 234 zusammen mit dem verschlüsselten Datenobjekt und dem verschlüsselten symmetrischen Schlüssel abgespeichert.

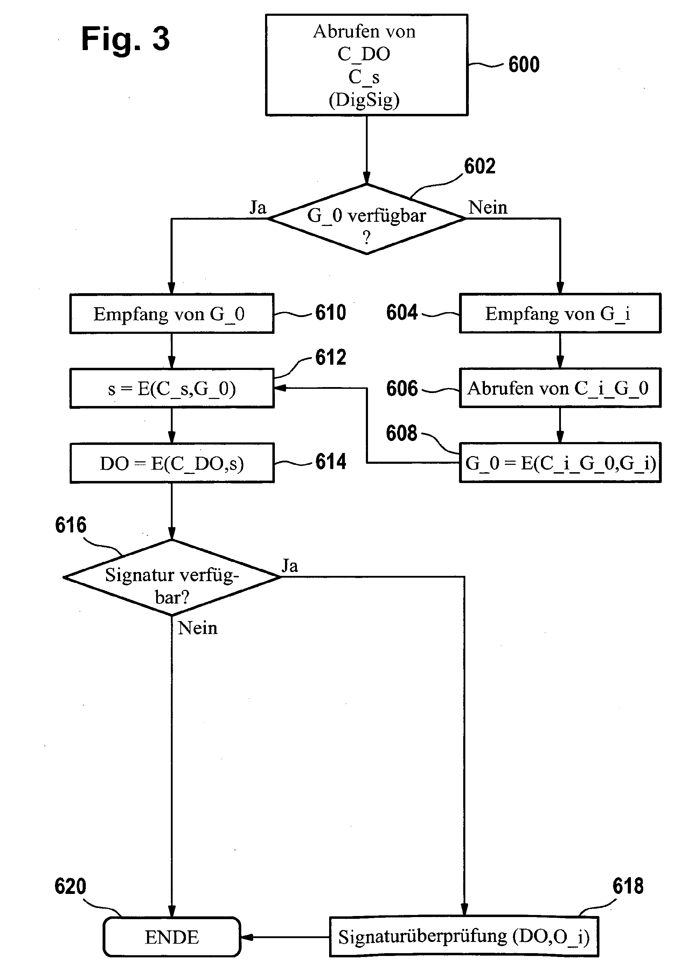

Die Figur 3 zeigt ein Flussdiagramm zum Entschlüsseln von Datenobjekten. Im Folgenden sei angenommen, dass dem Datenobjekt eine digitale Signatur hinzugefügt wurde. Damit erfolgt in Schritt 600 das Abrufen des verschlüsselten Datenobjektes, des verschlüsselten symmetrischen Schlüssels und der digitalen Signatur. In Schritt 602 wird überprüft, ob der initiale private Berechtigungsschlüssel verfügbar ist. Dies kann beispielsweise dann der Fall sein, wenn außer dem initialen Berechtigungsschlüssel keine weiteren Schlüssel in einer Sequenz ausgegeben wurden. Ist dies der Fall, wird in Schritt 610 dieser initiale private Berechtigungsschlüssel empfangen und in Schritt 612 erfolgt eine Entschlüsselung des verschlüsselten symmetrischen Datenobjektschlüssels mittels des initialen privaten Berechtigungsschlüssels. Dies ist möglich, da der symmetrische Datenobjektschlüssel s zuvor mit dem initialen öffentlichen Berechtigungsschlüssel O₀ verschlüsselt wurde, vgl. hierzu Schritt 230 der Figur 2.

Nachdem in Schritt 612 der symmetrische Schlüssel entschlüsselt wurde, erfolgt daraufhin in Schritt 614 eine Entschlüsselung des Datenobjekts mit dem so erhaltenen symmetrischen Schlüssel s. In Schritt 616 wird überprüft, ob zu dem Datenobjekt eine Signatur verfügbar ist. Ist dies der Fall, erfolgt in Schritt 618 eine Signaturüberprüfung unter Verwendung des öffentlichen Berechtigungsschlüssels der Chipkarte, welche ursprünglich zur Signierung des Datenobjektes herangezogen wurde. Hierzu ist nicht zwingenderweise das Vorliegen dieser Chipkarte selbst notwendig, da der öffentliche Berechtigungsschlüssel dieser Chipkarte ohne Weiteres auf einem öffentlichen Server abgelegt sein kann.

Ergibt sich in Schritt 602, dass der initiale private Berechtigungsschlüssel nicht verfügbar ist, typischerweise weil bereits eine Sequenz von zusätzlichen Berechtigungsschlüsseln bereitgestellt wurde, wird unter Verwendung eines beliebigen dieser Berechtigungsschlüsselpaare, welche augenblicklich zur Verfügung stehen, das Verfahren in Schritt 604 fortgesetzt. Im Folgenden sei angenommen, dass es sich hierbei um das Berechtigungsschlüsselpaar mit Index i handelt. Das Berechtigungsschlüsselpaar mit Index i umfasst einen privaten Berechtigungsschlüssel Gᵢ, welcher in Schritt 604 empfangen wird. Daraufhin wird in Schritt 606 entweder aus der Chipkarte selbst, welche Träger des privaten Berechtigungsschlüssels i ist, ein entsprechendes diesem Index zugeordnetes Chiffrat C_{i_G0} abgerufen, oder das Abrufen dieses Chiffrats erfolgt in Schritt 606 von einer externen Datenbank. Daraufhin erfolgt in Schritt 608 eine Entschlüsselung dieses Chiffrats mit dem in Schritt 604 empfangenen privaten Berechtigungsschlüssels Gᵢ. Dadurch erhält man in Schritt 608 den initialen privaten Berechtigungsschlüssel G₀. Dieser initiale private Berechtigungsschlüssel G₀ kann daraufhin in Schritt 612 dazu verwendet werden, den verschlüsselten symmetrischen Schlüssel zu entschlüsseln. Anschließend folgen, wie bereits angesprochen, die Schritte 614 bis 620, wobei in Schritt 620 das Verfahren endet.

Es sei hier noch angemerkt, dass in den Schritten 610 und 604 vom "Empfang" von privaten Schlüsseln die Rede ist. Dies soll jedoch nicht so verstanden werden, dass eine Übertragung der privaten Schlüssel über ein Medium, wie zum Beispiel ein Netzwerk, stattfindet. Vielmehr sollten vorzugsweise die Schritte 604 bis 612 bzw. 610 und 612 auf einer Chipkarte bzw. in einem Datenverarbeitungssystem selbst erfolgen, ohne dass ein unbefugter Benutzer dazu in der Lage ist, G₀ oder Gᵢ in irgendeiner Weise abzufangen bzw. auszulesen.

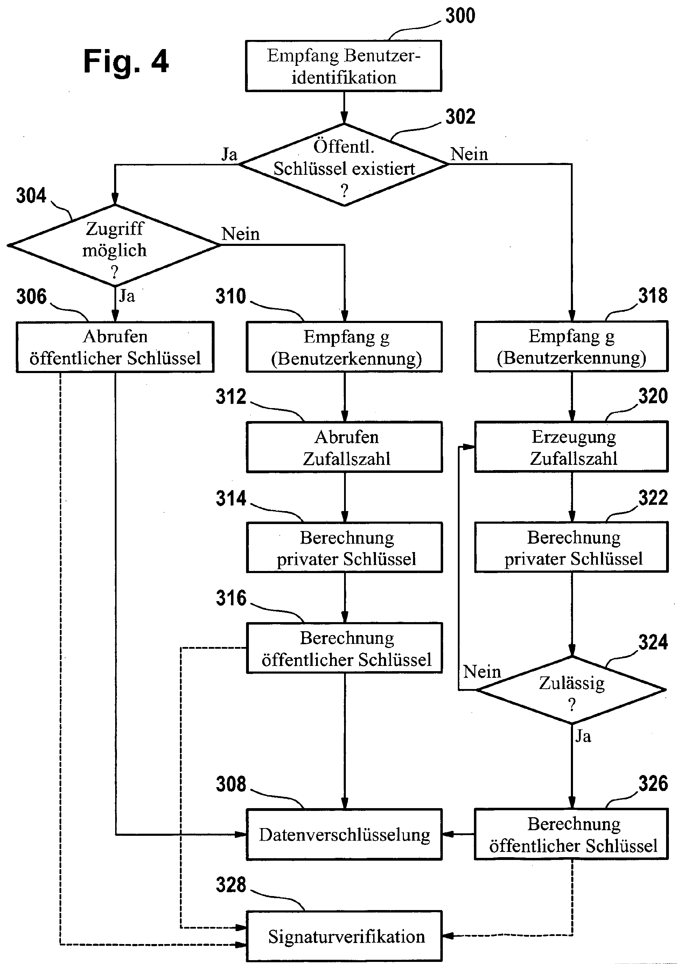

Die Figur 4 zeigt das Verfahren zur Erzeugung eines asymmetrischen Schlüsselpaares und seiner beispielhaften Verwendung zur Verschlüsselung von Datenobjekten und zur Verifikation digitaler Signaturen von Datenobjekten. In Schritt 300 wird eine eindeutige Benutzeridentifikation empfangen. Daraufhin erfolgt in Schritt 302 eine Überprüfung, ob ein öffentlicher Schlüssel existiert, welcher der in Schritt 300 empfangenen Benutzeridentifikation zugeordnet ist. Ist dies der Fall, wird in Schritt 304 überprüft, ob ein Zugriff auf diesen öffentlichen Schlüssel möglich ist. Ist dies möglich, wird in Schritt 306 der öffentliche Schlüssel abgerufen, und das Datenobjekt kann beispielsweise im Schritt 308 mittels des öffentlichen Schlüssels verschlüsselt werden oder es kann im Schritt 328 die digitale Signatur eines Datenobjekts verifiziert werden.

Ergibt die Prüfung in Schritt 304 hingegen, dass ein Zugriff auf den öffentlichen Schlüssel nicht möglich ist, dann muss der öffentliche Schlüssel erzeugt werden. Dies erfolgt beginnend mit Schritt 310, in welchem entweder eine frei wählbare Benutzerkennung empfangen und die Funktion g auf diese Benutzerkennung angewendet wird oder es wird bereits der Wert g(Benutzerkennung) empfangen. Daraufhin folgt Schritt 312, in welchem ein Zufallswert von einer entsprechenden Datenbank anhand der Benutzeridentifikation abgerufen wird. Durch Anwendung der Funktion f auf den Zufallswert und g(Benutzerkennung) erfolgt im Schritt 314 die Berechnung des privaten Schlüssels. Aus dem privaten Schlüssel wird schließlich in Schritt 316 der öffentliche Schlüssel berechnet, wobei der private und der öffentliche Schlüssel ein asymmetrisches kryptografisches Schlüsselpaar bilden.

Die Berechnung des öffentlichen Schlüssels im Schritt 316 erfolgt beispielsweise bei elliptischen Kurven dadurch, dass der öffentliche Schlüssel durch Multiplikation des privaten Schlüssels mit einem Kurvenpunkt einer elliptischen Kurve berechnet wird. Dem Verwender des Verschlüsselungsverfahrens der Figur 3 muss in diesem Fall ein Teil der zur Erzeugung des öffentlichen Schlüssels verwendeten Domainparameter bekannt sein.

Nach Berechnung des öffentlichen Schlüssels in Schritt 316 erfolgt wiederum in Schritt 308 eine Verschlüsselung des Datenobjektes mittels des öffentlichen Schlüssels oder in Schritt 328 die Verifikation einer digitalen Signatur eines Datenobjekts.

Ergibt sich im Prüfschritt 302, dass kein öffentlicher Schlüssel existiert, so erfordert dies eine initiale Erzeugung eines asymmetrischen Schlüsselpaars. Dies geschieht, indem in Schritt 318 entweder eine frei wählbare Benutzerkennung empfangen und die Funktion g auf diese Benutzerkennung angewendet wird oder es wird bereits der Wert g(Benutzerkennung) empfangen.

Daraufhin erfolgt in Schritt 320 die Erzeugung einer Zufallszahl, woraufhin in Schritt 322, wie bereits für Schritt 314 beschrieben, ein Kandidat für einen privaten Schlüssel erzeugt wird, indem die Funktion f auf g(Benutzerkennung) und die Zufallszahl angewendet wird.

In Schritt 324 erfolgt eine Zulässigkeitsprüfung, welche beispielsweise im Falle des elliptischen Kurvenverfahrens aus der Prüfung, ob der private Datenobjektschlüssel größer als 1 und kleiner als die Ordnung der elliptischen Kurve ist, besteht. Wird die Zulässigkeitsprüfung bestanden, so sind in Schritt 324 der Zufallswert sowie der private Schlüssel zulässig. Daraufhin kann in Schritt 326 die Berechnung des öffentlichen Schlüssels durchgeführt werden, worauf beispielsweise in Schritt 308 die Datenverschlüsselung oder in Schritt 328 die Verifikation der digitalen Signatur erfolgt.

Stellt sich hingegen in Schritt 324 heraus, dass der in Schritt 322 berechnete Kandidat für einen privaten Datenobjektschlüssel nicht zulässig ist, dann wird in Schritt 320 erneut eine Zufallszahl erzeugt, woraufhin die Schritte 322 und 324 wiederum durchgeführt werden. Dies geschieht so lange, bis die Zulässigkeitsprüfung in Schritt 324 erfolgreich ist, woraufhin der Schritt 326 mit der Berechnung des öffentlichen Schlüssels und beispielsweise der Schritt 308 mit der Datenverschlüsselung oder der Schritt 328 mit der Verifikation der digitalen Signatur durchgeführt werden.

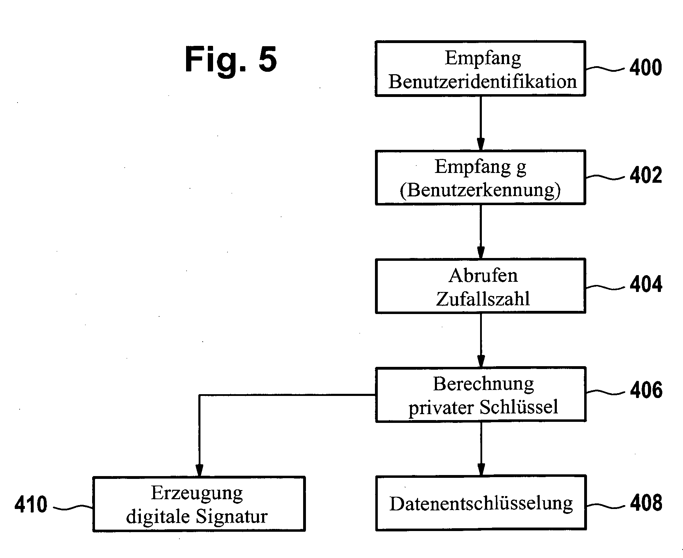

In der Figur 5 ist nun angenommen, dass, wie in der Figur 4 gezeigt, in Schritt 322 der private Schlüssel berechnet wurde und in Schritt 326 beispielsweise unter Verwendung einer elliptischen Kurve der öffentliche Schlüssel berechnet wurde. Die Figur 5 zeigt ein Verfahren zur Entschlüsselung eines Datenobjekts. Das Verfahren beginnt wiederum mit Schritt 400, dem Empfang einer eindeutigen Benutzeridentifikation. Außerdem wird daraufhin in Schritt 402 ein HASH-Wert einer Benutzerkennung empfangen. Unter Verwendung der Benutzeridentifikation wird in Schritt 404 von einer externen Datenbank eine Zufallszahl abgerufen, woraus unter Verwendung des HASH-Werts der Benutzerkennung in Schritt 406 ein privater Schlüssel berechnet werden kann. Dieser private Schlüssel kann nun dazu verwendet werden, um die mit dem öffentlichen Datenobjektschlüssel verschlüsselten Daten in Schritt 408 zu entschlüsseln.

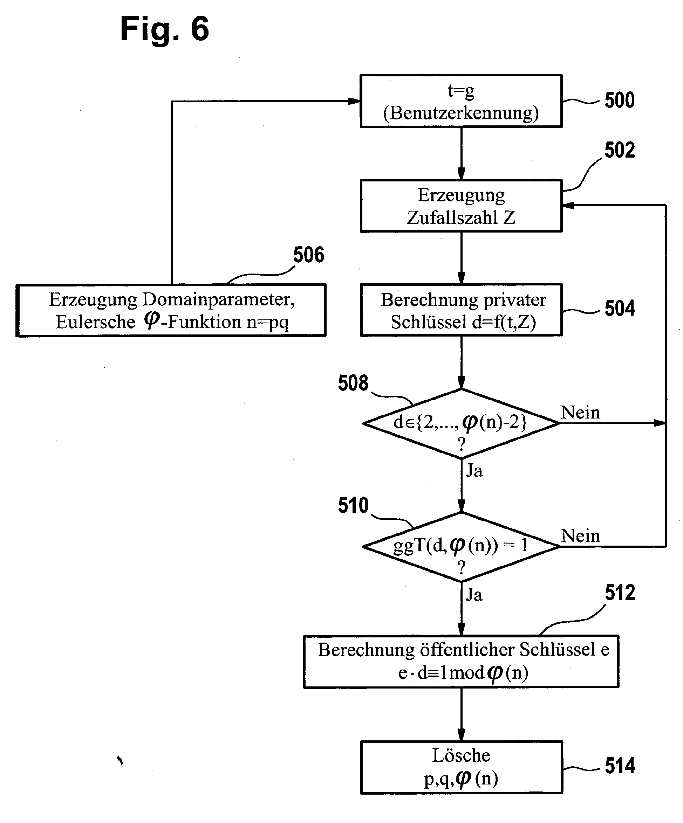

Die Figur 6 zeigt ein Flussdiagramm eines Verfahrens zur Berechung eines asymmetrischen Schlüsselpaares nach dem RSA-Verfahren. Das Verfahren beginnt mit Schritt 506, in dem zwei Primzahlen p und q gemäß den aktuellen Sicherheitsanforderungen für RSA gewählt werden. Die beiden Zahlen werden miteinander multipliziert und das Ergebnis n genannt. Auf n wird anschließend die Eulersche ϕ-Funktion angewendet, so dass man den Wert ϕ(n) erhält.

Im folgenden Schritt 500 wird eine Benutzerkennung empfangen, auf die die Funktion g angewendet wird, oder es wird direkt der Funktionswert g(Benutzerkennung) empfangen. Darauf folgt Schritt 502, in welchem ein Zufallswert erzeugt wird. Durch Anwendung der Funktion f auf den Zufallswert und g(Benutzerkennung) erfolgt im Schritt 504 die Berechnung eines Kandidaten für den privaten Schlüssel d.

In Schritt 508 erfolgt eine Zulässigkeitsprüfung, wobei die Zulässigkeitsprüfung im Falle des RSA Verfahrens mehrstufig verläuft. So wird zunächst in Schritt 508 geprüft, ob d im Intervall [2, ϕ(n) - 2] liegt.

Ergibt der Prüfschritt 508, dass die Prüfbedingung nicht erfüllt ist, springt das Verfahren zu Schritt 502 zurück, wo ein neuer Zufallswert erzeugt wird. Daraufhin folgen wiederum die Schritte 504 mit dem erneuten Berechnen eines Kandidaten für den privaten Schlüssel e anhand des neuen Zufallswerts und dem erneuten Prüfen des Kandidaten d in Schritt 508. Diese Schleife der Schritte 502, 504 und 508 wiederholt sich so lange, bis die Prüfbedingung in Schritt 508 erfüllt ist. Erst dann setzt sich das Verfahren mit Schritt 510 fort.

Schritt 510 umfasst einen weiteren Prüfschritt, nämlich ob ϕ(n) und der Schlüsselkandidat d teilerfremd sind, d.h. ggT(d, ϕ(n)) = 1. Ist dies nicht der Fall, springt das Verfahren wieder zurück zu Schritt 502 und ein neuer Zufallswert wird erzeugt, gefolgt von den Schritten 504, 508, 510. Auch diese Schleife der Schritte 502, 504, 508 und 510 wiederholt sich so lange, bis die Prüfbedingung in Schritt 510 erfüllt ist. Erst dann setzt sich das Verfahren mit Schritt 512 fort. Vorzugsweise wird der Prüfschritt 508 vor dem Prüfschritt 510 durchgeführt, da der Rechenaufwand für Schritt 508 wesentlich geringer ist als der Rechenaufwand für Schritt 510.

In Schritt 512 erfolgt schließlich die Berechnung des öffentlichen Schlüssels e, so dass e die Kongruenz-Beziehung e · d ≡ 1 mod ϕ(n) mit e ∈ [1, ϕ(n) - 1] erfüllt. Zur Verwendung des privaten Schlüssels d in kryptografischen Verfahren muss auch n bekannt gegeben werden.

Im abschließenden Schritt 514 werden die Zahlen p, q, und ϕ (n) verworfen, d.h. gelöscht.

Neben den Prüfschritten 508 und 510 können weitere Prüfschritte zum Einsatz kommen, um so die Sicherheit des erzeugten asymmetrischen Schlüsselpaares zu erhöhen.

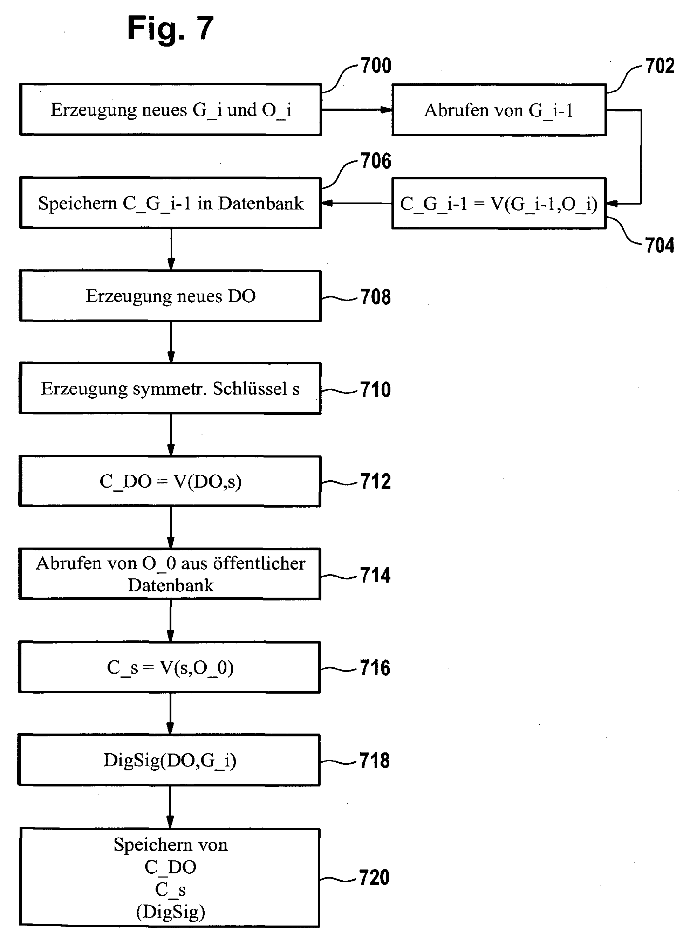

Die Figur 7 zeigt ein weiteres Flussdiagramm eines Verfahrens zur hierarchischen Bereitstellung von Berechtigungsschlüsseln und zur Verschlüsselung von Daten. Das Verfahren beginnt in dem Schritt 700, in welchem ein neuer privater Schlüssel Gi und ein neuer öffentlicher Schlüssel Oi erzeugt werden. Der neue private Schlüssel Gi und der neue öffentliche Schlüssel Oi können dabei auf einer neuen Chipkarte gespeichert sein. Daraufhin erfolgt in Schritt 702 das Abrufen eines privaten Schlüssels Gi-1, welcher in der Sequenz von Berechtigungsschlüsseln dem Paar von Berechtigungsschlüsseln unmittelbar vorangeht, welches in Schritt 700 erzeugt wurde. In einem praktischen Beispiel von Chipkarten würde in Schritt 700 das neue Schlüsselpaar Gi und Oi von einer neuen Chipkarte abgerufen, wohingegen in Schritt 702 der private Vorgänger-Berechtigungsschlüssel Gi-1 von einer Vorgänger-Chipkarte abgerufen wird. Daraufhin wird in Schritt 704 dieser private Vorgänger-Schlüssel Gi-1 mit dem öffentlichen Schlüssel Oi der neuen Chipkarte verschlüsselt. Das sich so ergebende Chiffrat wird in Schritt 706 in einer Datenbank abgelegt.

Nachdem in den Schritten 700 bis 706 eine hierarchische Bereitstellung von Berechtigungsschlüsseln erfolgt ist, folgt in den Schritten 708 bis 720 ein Verschlüsselungsvorgang von Datenobjekten. In anderen Worten müssen die Schritte 700 bis 706 lediglich einmalig bei Ausgabe einer neuen Chipkarte mit neuen asymmetrischen Schlüsseln Gi und Oi durchgeführt werden, wohingegen die Schritte 708 bis 720 für jeden Verschlüsselungsvorgang von Datenobjekten durchgeführt werden müssen. In Schritt 708 wird nun ein neues Datenobjekt erzeugt. Daraufhin erfolgt in Schritt 710 die Erzeugung eines symmetrischen Schlüssels s, welcher in Schritt 712 dazu verwendet wird, um das Datenobjekt des Schrittes 708 zu verschlüsseln. In Schritt 714 erfolgt das Abrufen des initialen öffentlichen Berechtigungsschlüssels O0 aus einer öffentlichen Datenbank, wobei in Schritt 716 dieser initiale öffentliche Berechtigungsschlüssel zur Verschlüsselung des symmetrischen Schlüssels s, welcher in Schritt 710 erhalten wurde, verwendet wird.

Schließlich erfolgt eine Speicherung des verschlüsselten Datenobjekts und des verschlüsselten symmetrischen Schlüssels in einer entsprechenden Patientendatenbank in Schritt 720.

Der Schritt 718, welcher nach Schritt 716 folgt, ist ein optionaler Schritt, in welchem die Möglichkeit besteht, das Datenobjekt zusätzlich zu signieren, um so einem unbefugten Missbrauch und einer Manipulation des Datenobjekts vorzubeugen. Eine solche digitale Signierung erfolgt vorzugsweise mit dem privaten Berechtigungsschlüssel Gi der aktuell verwendeten Chipkarte. Wurde der Schritt 718 durchgeführt, wird die digitale Signatur zusätzlich in Schritt 720 zusammen mit dem verschlüsselten Datenobjekt und dem verschlüsselten symmetrischen Schlüssel abgespeichert.

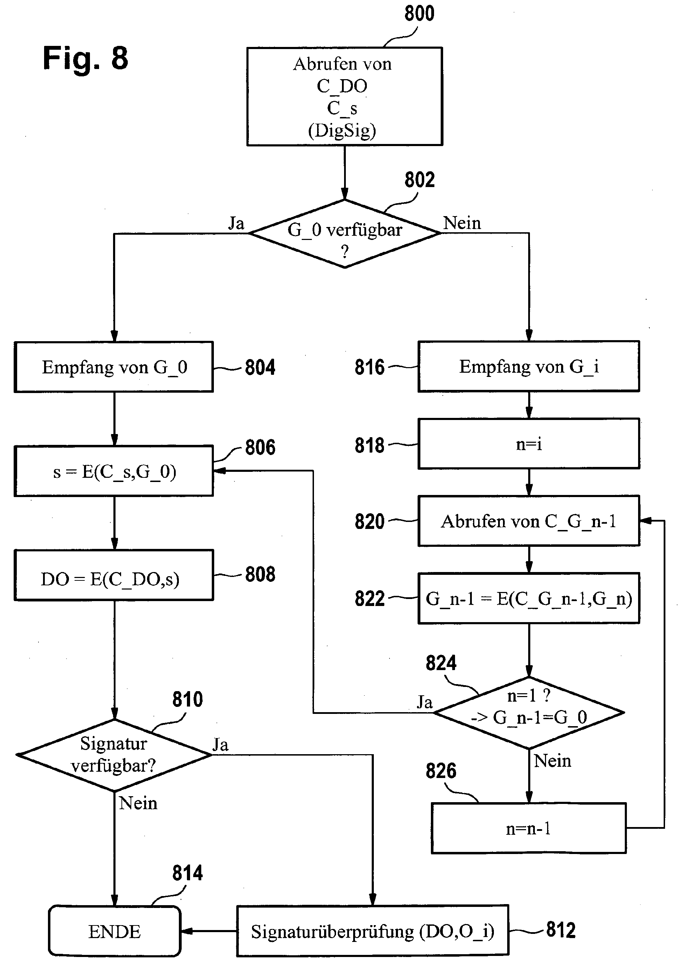

Die Figur 8 zeigt ein weiteres Flussdiagramm zum Entschlüsseln von Datenobjekten. Im Folgenden sei angenommen, dass dem Datenobjekt eine digitale Signatur zugefügt wurde. Damit erfolgt in Schritt 800 das Abrufen des verschlüsselten Datenobjekts, des verschlüsselten symmetrischen Schlüssels und der digitalen Signatur. In Schritt 802 wird überprüft, ob der initiale private Berechtigungsschlüssel verfügbar ist. Dies kann beispielsweise dann der Fall sein, wenn außer dem initialen Berechtigungsschlüssel keine weiteren Schlüssel in einer hierarchischen Sequenz ausgegeben wurden. Ist dies der Fall, wird in Schritt 804 dieser initiale private Berechtigungsschlüssel G0 empfangen und in Schritt 806 erfolgt eine Entschlüsselung des verschlüsselten symmetrischen Datenobjektschlüssels mittels des initialen privaten Berechtigungsschlüssels G0. Dies ist möglich, dass der symmetrische Datenobjektschlüssel s zuvor mit dem initialen öffentlichen Berechtigungsschlüssel O0 verschlüsselt wurde, vgl. hierzu Schritt 716 der Figur 7.

Nachdem in Schritt 806 der symmetrische Schlüssel entschlüsselt wurde, erfolgt daraufhin in Schritt 808 eine Entschlüsselung des Datenobjekts mit dem so erhaltenen symmetrischen Schlüssel s. In Schritt 810 wird überprüft, ob zu dem Datenobjekt eine Signatur verfügbar ist. Ist dies der Fall, erfolgt in Schritt 812 eine Signaturüberprüfung unter Verwendung des öffentlichen Berechtigungsschlüssels der Chipkarte, welche ursprünglich zur Signierung des Datenobjekts herangezogen wurde. Hierzu ist nicht notwendigerweise das Vorliegen dieser Chipkarte selbst notwendig, da der öffentliche Berechtigungsschlüssel dieser Chipkarte ohne Weiteres auf einem öffentlichen Server abgelegt sein kann.

Ergibt sich in Schritt 802, dass der initiale Berechtigungsschlüssel nicht direkt verfügbar ist, typischerweise weil bereits eine Sequenz von zusätzlichen Berechtigungsschlüsseln bereitgestellt wurde, wird unter Verwendung eines beliebigen dieser Berechtigungsschlüsselpaare, welche augenblicklich zur Verfügung stehen, das Verfahren in Schritt 816 fortgesetzt. Im Folgenden sei angenommen, dass es sich bei dem zur Entschlüsselung des Datenobjekts verfügbaren Berechtigungsschlüsselpaar um das Schlüsselpaar mit Index i handelt. Das Berechtigungsschlüsselpaar mit Index i umfasst einen privaten Berechtigungsschlüssel Gi, welcher in Schritt 816 empfangen wird. Darauf wird in Schritt 818 ein Zählindex n=i gesetzt. Daraufhin folgt Schritt 820 mit dem Abrufen eines Chiffrats, welches zuvor bei Aktivierung der Chipkarte mit dem neuen Satz von Berechtigungsschlüsseln mit Index i erzeugt wurde, von einer externen Datenbank. Bei diesem Chiffrat, welches in Schritt 820 abgerufen wird, handelt es sich um das Chiffrat, welches in der Figur 7 in Schritt 704 erzeugt wurde und in Schritt 706 in dieser Datenbank abgespeichert wurde.

Nachdem nun in Schritt 820 das Chiffrat von der Datenbank abgerufen wurde, erfolgt in Schritt 822 das Entschlüsseln des Chiffrats mit dem gegenwärtig verfügbaren privaten Berechtigungsschlüssel mit Index n, d. h. im vorliegenden Schritt Index n=i. In anderen Worten wird der private Berechtigungsschlüssel der zur Entschlüsselung verwendeten Chipkarte dazu verwendet, um das Chiffrat zu entschlüsseln. Dieser Entschlüsselungsvorgang in Schritt 822 liefert den privaten Berechtigungsschlüssel mit Index n-1, d. h. Index i-1, oder in anderen Worten der private Berechtigungsschlüssel der Vorgänger-Chipkarte in der Sequenz von Chipkarten, welche der Chipkarte mit Index i unmittelbar vorangeht.

In Schritt 824 wird überprüft, ob der Laufindex n=1 ist. In diesem Fall würde nämlich der private Berechtigungsschlüssel, welcher in Schritt 822 aus dem Chiffrat extrahiert wurde, dem initialen privaten Berechtigungsschlüssel G0 entsprechen. Ist dies der Fall, erfolgt nach Schritt 824 die Durchführung der Schritte 806 bis 814, wie obig beschrieben.

Im Folgenden sei jedoch angenommen, dass sich in Schritt 824 ergibt, dass n größer als 1 ist, sodass nach Schritt 824 der Schritt 826 mit der Erniedrigung des Laufindex n um 1 erfolgt, d.h. n=i-2. Nach Schritt 826 wiederholt sich das Verfahren der Schritte 820 bis 824 wie obig beschrieben.

Dies bedeutet, dass in Schritt 820 nun das Chiffrat von der externen Datenbank abgerufen wird, welches dem Index i-2 zugeordnet ist. Unter Verwendung des in der vorigen Schleife erhaltenen privaten Berechtigungsschlüssels mit Index i-1 lässt sich nun das soeben in Schritt 820 abgerufene Chiffrat wiederum entschlüsseln, woraus in Schritt 822 der private Berechtigungsschlüssel mit Index i-2 erhalten wird. Dem folgt wiederum Schritt 824 mit der Überprüfung, ob der Laufindex 1 beträgt, d. h. ob der in Schritt 822 erhaltene private Berechtigungsschlüssel dem initialen privaten Berechtigungsschlüssel G0 entspricht. Ist dies der Fall, folgt Schritt 806, wohingegen bei Nichterfüllung dieser Bedingung Schritt 826 mit der Erniedrigung des Laufindex n um 1 erfolgt.

Somit wird durch Durchführung der Schritte 820 bis 826 in rekursiver Weise unter sequenziellem Abrufen von entsprechenden Chiffraten der initiale private Berechtigungsschlüssel G0 extrahiert, um damit eine Entschlüsselung des verschlüsselten symmetrischen Schlüssels vorzunehmen.

### Bezugszeichenliste

- 100: Datenverarbeitungssystem
- 102: Eingabemittel
- 104: Bildschirm
- 106: Schnittstelle
- 108: Prozessor
- 110: Speicher
- 112: Applet
- 114: Modul
- 116: Modul
- 118: Modul
- 120: Netzwerk
- 122: Datenbank
- 124: Benutzeridentifikation
- 126: öffentlicher Schlüssel
- 128: Zufallszahl
- 130: verschlüsseltes Datenobjekt
- 132: Datenbank
- 134: Datenbank
- 150: Chiffrat
- 152: Chipkarte
- 154: privater Schlüssel
- 156: öffentlicher Schlüssel
- 158: Hardwaremodul
- 160: Chipkarte
- 162: privater Schlüssel
- 164: öffentlicher Schlüssel
- 166: Hardwaremodul
- 200: Applet
- 202: Modul
- 204: Modul
- 206: Modul
- 208: Datenverarbeitungssystem
- 210: Schnittstelle
- 212: Prozessor
- 214: Speicher
- 216: Programm
- 218: Modul
- 220: Modul
- 222: Modul

## Patentansprüche

1. Computerimplementiertes Verfahren (114; 116) zur Entschlüsselung eines Datenobjekts (130), wobei das Datenobjekt über einen initalen ersten Berechtigungsschlüssel entschlüsselbar ist, wobei der initale erste Berechtigungsschlüssel (G₀) zusammen mit einem initialen zweiten Berechtigungsschlüssel (O₀) ein initales asymmetrisches kryptografisches Schlüsselpaar ({G₀, O₀}) bildet, wobei das initiale asymmetrische Schlüsselpaar ({G₀, O₀}) Teil einer Schlüsselpaarsequenz ({G₀, O₀}_{;} {G₁, O₁}; ...; {Gᵢ, Oᵢ}) ist, die neben dem initialen asymmetrischen Schlüsselpaar ({G₀, O₀}) noch wenigstens ein weiteres asymmetrisches Schlüsselpaar ({Gᵢ, Oᵢ}) aufweist, wobei das Verfahren die folgenden Schritte umfasst:
- Zugriff auf einen ersten Berechtigungsschlüssel (154), wobei der erste Berechtigungsschlüssel zusammen mit einem zweiten Berechtigungsschlüssel (156) ein asymmetrisches kryptografisches Schlüsselpaar (154, 156) bildet, wobei das asymmetrische Schlüsselpaar (154, 156) Teil der Schlüsselpaarsequenz ({G₀, O₀};{G₁, O₁}; ... ; {Gᵢ, Oᵢ}) ist,
- Abrufen (606) eines Chiffrats (150), wobei das Chiffrat dem asymmetrischen kryptografischen Schlüsselpaar (154,156) zugeordnet ist, wobei das Chiffrat den initialen ersten Schlüssel (G₀) verschlüsselt mit dem zweiten Berechtigungsschlüssel (156) umfasst,
- Entschlüsseln (608) des verschlüsselten initialen ersten Schlüssels (G₀) mit dem ersten Berechtigungsschlüssel (154),
- Entschlüsseln (614) des verschlüsselten Datenobjekts (130) über den entschlüsselten initialen ersten Schlüssel (G₀).

2. Verfahren nach Anspruch 1 mit den zusätzlichen Schritten:
- Durchführung des Schritts einer Signaturüberprüfung (618) des Datenobjekts, wobei die Signaturüberprüfung die Schritte umfasst:
- Lesen einer dem Datenobjekt zugeordneten Signatur,
- Verifizierung der Signatur des Datenobjekts, wobei die Verifikation mit dem zweiten Berechtigungsschlüssel erfolgt.

3. Verfahren nach einem der vorhergehenden Ansprüche, ferner mit Instruktionen zur Durchführung des Schritts des Empfangens einer dem asymmetrischen kryptografischen Schlüsselpaar zugeordneten Schlüsselpaarkennung, wobei das Chiffrat (150) anhand der Schlüsselpaarkennung abgerufen wird.

4. Verfahren nach einem der vorhergehenden Ansprüche, wobei das Chiffrat (150) von einer Datenbank (122) abgerufen wird.

5. Verfahren nach einem der vorhergehenden Ansprüche, wobei das asymmetrische Schlüsselpaar zusammen mit dem Chiffrat (150) auf einem tragbaren Datenträger (152; 160) gespeichert ist.

6. Verfahren nach einem der vorigen Ansprüche, wobei das Datenobjekt mit einem symmetrischen Datenobjektschlüssel verschlüsselt ist, wobei der symmetrische Datenobjektschlüssel mit dem initalen zweiten Berechtigungsschlüssel verschlüsselt ist, wobei das Entschlüsseln des verschlüsselten Datenobjekts die folgenden weiteren Schritte umfasst:
- Entschlüsseln des verschlüsselten symmetrischen Datenobjektschlüssels mit dem entschlüsselten initialen ersten Berechtigungsschlüssel,
- Entschlüsseln des verschlüsselten Datenobjekts mit dem entschlüsselten symmetrischen Schlüssel.

7. Verfahren nach einem der vorigen Ansprüche, wobei der Zugriff auf den ersten Berechtigungsschlüssel die folgenden Schritte umfasst:
- Empfang einer Benutzeridentifikation (124) und einer der Benutzeridentifikation (124) zugeordneten Benutzerkennung,
- Abrufen eines der Benutzeridentifikation (124) zugeordneten Zufallswertes von einer weiteren Datenbank,
- Berechnen des ersten Berechtigungsschlüssels, wobei in die Berechnung der Zufallswert (128) und die Benutzerkennung eingehen.

8. Verfahren nach Anspruch 7, wobei die Signaturüberprüfung ferner den Schritt umfasst des Berechnens des zweiten Berechtigungsschlüssels aus dem ersten Berechtigungsschlüssel mittels eines asymmetrischen kryptografischen Schlüsselerzeugungsverfahrens, wobei der erste und der zweite Berechtigungsschlüssel das asymmetrische kryptografische Schlüsselpaar bilden.

9. Verfahren nach Anspruch 7 oder 8, wobei der Zufallswert (128) über eine sichere Kommunikationsverbindung von der weiteren Datenbank (132) abgerufen wird.

10. Verfahren nach einem der vorigen Ansprüche 8 bis 9, wobei der Zufallswert (128) verschlüsselt in der weiteren Datenbank (132) abgespeichert ist.

11. Computerprogrammprodukt mit ausführbaren Programminstruktionen zur Durchführung eines Verfahrens nach einem der vorigen Ansprüche.

## Claims

1. A computer-implemented process (114; 116) for decryption of a data object (130), wherein the data object can be decrypted through an initial first authorization key, the initial first authorization key (G₀) together with an initial second authorization key (O₀) forming an initial asymmetric cryptographic key pair ({G₀, O₀}), the initial asymmetric key pair ({G₀, O₀}) being part of a sequence of key pairs ({G₀, O₀); {G₁, O₁}; ... {Gᵢ, Oᵢ}), which has at least one other asymmetric key pair ({Gᵢ, Oᵢ)) besides the initial asymmetric key pair ({G₀, O₀}), the process comprising the following steps:
- Accessing a first authorization key (154), the first authorization key together with a second authorization key (156) forming an asymmetric cryptographic key pair (154, 156), the asymmetric key pair (154, 156) being part of the sequence of key pairs ({G₀, O₀}; {G₁, O₁}; ... {Gᵢ, Oᵢ});
- Retrieving (606) a ciphertext (150), the ciphertext being associated with the asymmetric cryptographic key pair (154, 156), the ciphertext comprising the initial first key (G₀) encrypted with the second authorization key (155);
- Decrypting (608) the encrypted initial first key (G₀) with the first authorization key (154);
- Decrypting (614) the encrypted data object (130) through the decrypted initial first key (G₀).

2. The process described in claim 1 with the additional steps:
- Performing the step of signature verification (618) of the data object, the signature verification comprising the steps:
- Reading a signature associated with the data object;
- Verifying the signature of the data object, the verification being done with the second authorization key.

3. The process described in one of the preceding claims, further comprising instructions for performing the step of receiving a key pair ID associated with the asymmetric cryptographic key pair, the ciphertext (150) being retrieved using the key pair ID.

4. The process described in one of the preceding claims, wherein the ciphertext (150) is retrieved from a database (122).

5. The process described in one of the preceding claims, wherein the asymmetric key pair is stored together with the ciphertext (150) on a portable data carrier (152; 160).

6. The process described in one of the preceding claims, wherein the data object is encrypted with a symmetric data object key, the symmetric data object key being encrypted with the initial second authorization key, the decryption of the encrypted data object comprising the following other steps:
- Decryption of the encrypted symmetric data object key with the decrypted initial first authorization key;
- Decryption of the encrypted data object with the decrypted symmetric key.

7. The process described in one of the preceding claims, wherein accessing the first authorization key comprises the following steps:
- Receiving a user identification (124) and an user ID associated with the user identification (124);
- Retrieving a random value associated with the user identification (124) from another database;
- Calculating the first authorization key, the calculation involving the random value (128) and the user ID.

8. The process described in claim 7, wherein the signature verification comprises the further step of calculating the second authorization key from the first authorization key by means of an asymmetric cryptographic key production process, wherein the first and second authorization keys form the asymmetric cryptographic key pair.

9. The process described in claim 7 or 8, wherein the random value (128) is retrieved from the other database (132) over a secure communications link.

10. The process described in one of the preceding claims 8 through 9, wherein the random value (128) is saved in the other database (132) in encrypted form.

11. A computer program product with executable program instructions to perform of a process described in one of the preceding claims.

## Revendications

1. Procédé mis en oeuvre par ordinateur (114 ; 116) pour le décryptage d'un objet de données (130), où l'objet de données peut être décrypté par l'intermédiaire d'une première clé d'autorisation initiale, où la première clé d'autorisation initiale (G₀) forme, ensemble avec une seconde clé d'autorisation initiale (O₀), une paire de clés (G₀, O₀) cryptographiques asymétrique initiale, où la paire de clés (G₀, O₀) asymétrique initiale est une composante d'une séquence de paires de clés ({G₀,O₀} ; {G₁, O₁} ; ; {Gᵢ, Oᵢ}), qui présente, conjointement à la paire de clés asymétrique initiale ({G₀, O₀}), encore au moins une autre paire de clés asymétrique ({Gᵢ, Oᵢ}) ; où le procédé comprend les étapes suivantes :
- accès à une première clé d'autorisation (154), où la première clé d'autorisation, ensemble avec une seconde clé d'autorisation (156), forme une paire de clés (154, 156) cryptographique asymétrique, où la paire de clés (154, 156) asymétrique est une composante de la séquence de paires de clés ({G₀,O₀} ; {G₁, O₁} ; ; {Gᵢ, Oᵢ}),
- récupération (606) d'un texte chiffré (150), où le texte chiffré est associé à la paire de clés (154, 156) cryptographique asymétrique, où le texte chiffré comprend la première clé initiale (G0) encodée avec la seconde clé d'autorisation (156),
- décryptage (608) de la première clé (G0) initiale encodée avec la première clé d'autorisation (154),
- décryptage (614) de l'objet de données (130) encodé par l'intermédiaire de la première clé (G0) initiale décryptée.

2. Procédé selon la revendication 1, avec les étapes supplémentaires :
- réalisation d'une étape de contrôle d'une signature (618) de l'objet de données, où le contrôle de la signature comprend les étapes de :
- lecture d'une signature associée à l'objet de données,
- vérification de la signature de l'objet de données, où la vérification est effectuée avec la seconde clé d'autorisation.

3. Procédé selon l'une des revendications précédentes, avec en outre des instructions pour la réalisation de l'étape de réception d'un identifiant de paires de clés associé à la paire de clés cryptographique asymétrique, où le texte chiffré (150) est récupéré à l'aide de l'identifiant de paire de clés,

4. Procédé selon l'une des revendications précédentes, où le texte chiffré (150) est récupéré à partir d'une banque de données (122).

5. Procédé selon l'une des revendications précédentes, où la paire de clés asymétrique, ensemble avec le texte chiffré (150), est enregistrée sur un support de donnée (152 ; 160) portatif.

6. Procédé selon l'une des revendications antérieures, où l'objet de données est encodé avec une clé d'objet de données symétrique, où la clé d'objet de données symétrique est encodée avec la seconde clé d'autorisation initiale, où le décryptage de l'objet de données encodé comprend les nouvelles étapes suivantes :
- décryptage de la clé d'objet de données symétrique encodée avec la première clé d'autorisation initiale,
- décryptage de l'objet de données encodé avec la clé symétrique décryptée.

7. Procédé selon l'une des revendications antérieures, où l'accès à la première clé d'autorisation comprend les étapes suivantes :
- réception d'une identification d'utilisateur (124) et d'un identifiant d'utilisateur associé à l'identification d'utilisateur (124),
- récupération d'une valeur aléatoire associée à l'identification d'utilisateur (124) à partir d'une banque de données,
- calcul de la première clé d'autorisation, où la valeur aléatoire (128) et l'identifiant d'utilisateur sont impliqués dans le calcul.

8. Procédé selon la revendication 7, où le contrôle de la signature comprend en outre l'étape du calcul de la seconde clé d'autorisation à partir de la première clé d'autorisation au moyen d'un procédé de génération de clés cryptographiques asymétriques, où les première et seconde clés d'autorisation forment la paire de clés cryptographique asymétrique.

9. Procédé selon les revendications 7 ou 8, où la valeur aléatoire (128) est récupérée à partir d'une autre banque de données (132) par l'intermédiaire d'une liaison de communication sécurisée.

10. Procédé selon l'une des revendications antérieures 8 à 9, où la valeur aléatoire (128) est enregistrée encodée dans une autre banque de données (132).

11. Produit d'un programme d'ordinateur avec des instructions de programme exécutables pour la réalisation d'un procédé selon l'une des revendications antérieures.
